# EUROPEAN PATENT APPLICATION

(11) **EP 4 813 387 A1**
(43) Date of publication of application: **30.09.2026**
(21) Application number: 25166187.2
(22) Date of filing: 25.03.2025
(51) Int. Cl.: A61K 31/4545, A61K 31/47, A61P 37/08

(54) **COMBINATION OF RUPATADINE AND MONTELUKAST FOR USE IN THE TREATMENT OF SEASONAL ALLERGIC RHINITIS**

(71) Applicant: NOUCOR HEALTH, S.A., 08184 Barcelona (ES); Intas Pharmaceuticals Ltd., Ahmedabad - 380054, Gujarat (IN)
(72) Inventor: Barcos, Antonio Eloy Brusau, 08195 Sant Cugat del Valles (ES); González, Daniel Peris, 08172 Sant Cugat del Vallés (ES); Márquez, Xavier Formosa, 08039 Barcelona (ES)
(74) Representative: Elkington and Fife LLP

(57) **Abstract**

The present invention relates to a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by a single T4NSS measurement evaluation.

## Description

### FIELD OF THE INVENTION

The present invention relates to a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by a single T4NSS measurement evaluation.

### BACKGROUND OF THE INVENTION

### Allergic rhinitis

Allergic rhinitis (AR) is a heterogeneous disorder that despite its high prevalence is often undiagnosed. It is diagnosed by a detailed history, supported by examination of the patient as a whole as well as nasal symptoms, plus, if necessary, testing for allergen-specific immunoglobulin (Ig)E. Allergic rhinitis is a worldwide health problem characterised by one or more symptoms, including sneezing, itching, nasal congestion and rhinorrhea, which are often accompanied by ocular pruritus/redness and/or watery eyes in 60-70% of patients. Although not life-threatening, AR symptoms are frequently bothersome, adversely affecting work and quality of life of the affected individual and causing a significant burden on both the individual and society (Izquierdo-Dominguez et al., Current Allergy and Asthma Reports, 2013).

Allergic rhinitis is a major IgE-mediated chronic respiratory disorder, which has traditionally been subdivided, based on time of exposure, into seasonal and perennial disease. Seasonal allergic rhinitis (SAR) is usually caused by a wide variety of outdoor allergens such as pollen and moulds while perennial allergic rhinitis (PAR) is most frequently caused by indoor allergens, such as dust mites, animal dander, insects and moulds. Overall, approximately 20% of all cases are strictly seasonal, 40% perennial, and 40% mixed (perennial with seasonal exacerbations) (Passali et al., Asia Pac Allergy, 2018).

Tree, grass, and weed pollens and outdoor mould spores are common seasonal allergens. The symptoms typically appear during a defined season in which aeroallergens are abundant in the outdoor air. The length of seasonal exposure to these allergens is dependent on geographic location. Typical symptoms during pollen exposure include the symptoms described above, with explosive onset of profuse watery rhinorrhea, itching, and sneezing, along with frequent allergic symptoms of the eye. Congestion also occurs but usually is not the most troubling symptom. The onset and offset of symptoms usually track the seasonal pollen counts. However, hyperresponsiveness to irritant triggers, which develops from the inflammatory reaction of the late phase and priming responses, often persists after cessation of the pollen season. Such triggers include tobacco smoke, noxious odours, changes in temperature, and exercise (Skoner, Journal of Allergy and Clinical Immunology, 2001).

There is wide variation in the reported prevalence even within the same continent for AR. Recently, prevalence of AR ranged from 3.6% to 22.8% for Africa, from 3.5% to 54.5% for America, from 1.0% to 47.9% for Asia, from 1.0% to 43.9% for Europe, and from 19.2% to 47.5% for Oceania. In Europe, trends from Denmark, Finland, France, Germany, Italy, Russia, Scotland, and Sweden indicate an increase in the prevalence of AR over time (Savouré et al., Clin Transl Allergy, 2022).

Under normal conditions, the nasal mucosa quite efficiently humidifies and cleans inspired air. This is the result of orchestrated interactions of local and humoral mediators of host defence. In AR, these mechanisms go awry and contribute to the signs and symptoms of the disorder. Several mediators such as histamine, cysteinyl leukotrienes (CysLT), prostaglandins and kinins play an active role in the pathophysiology of AR. The tendency to develop IgE/mast cell/TH2 lymphocyte immune responses is inherited by atopic patients. Exposure to threshold concentrations of dust mite faecal proteins; cockroach allergen; cat, dog, and other danders; pollen grains; or other allergens for prolonged periods of time leads to the presentation of the allergen by antigen presenting cells to CD4⁺ T lymphocytes, which then release interleukin (IL)-3, IL-4, IL-5, and other TH2 cytokines. These cytokines drive proinflammatory processes, such as IgE production, against these allergens through the mucosal infiltration and actions of plasma cells, mast cells, and eosinophils. Once the patient has become sensitized to allergens, subsequent exposures trigger a cascade of events that result in the symptoms of AR.

Histamine is released from mast cells and basophils by antigenic stimulation causing smooth muscle contraction, increased vascular permeability and mucus formation. Platelet-activating factor (PAF) is an important mediator of AR as can be concluded from the effectiveness of the PAF antagonist ABT-491 in rat and guinea pig models of AR (Albert et al., Inflammation Research,1998). The biological properties of this mediator include vasodilation and an increase in vascular permeability that may contribute to the appearance of rhinorrhea and nasal congestion. Both PAF and its metabolite, lyso-PAF, have been detected in the nasal fluids and plasma of patients with rhinitis. Platelet-activating factor and histamine are known to complement each other *in vivo*; histamine is a mediator of early response, being released from preformed reservoirs in mast cells, whereas PAF is mainly synthesized de novo (Snyder, Clin Rev Allergy, 1994).

CysLT (LTC₄, LTD₄, LTE₄) are potent inflammatory eicosanoids released from various cells including mast cells and eosinophils. These mediators bind to CysLT receptors. The CysLT type-1 receptor is found in the human airway (including airway smooth muscle cells and airway macrophages) and on other pro-inflammatory cells (including eosinophils and certain myeloid stem cells. CysLTs and leukotriene receptor occupancy has been linked to several processes in AR, including: (1) dilation of nasal blood vessels and vascular permeability with oedema formation, both leading to nasal congestion, (2) increased mucus production and secretion, leading to rhinorrhea, and (3) recruitment of inflammatory cells from the bloodstream into tissue, thus perpetuating the inflammatory response. However, there is a growing body of evidence suggesting that CysLTs are multi-functional mediators playing a broader role in the inflammation that characterises allergic disorders such as AR.

### Current treatment guidelines and options for allergic rhinitis

The leading authority for the treatment of allergic rhinitis is The European Forum for Research and Education in Allergy and Airways diseases (EUFOREA). EUFOREA has the mission to implement optimal care for patients suffering from allergies and chronic respiratory conditions.

In 2020, a pocket guide for adult AR was developed by an extended global panel of EUFOREA experts, it included a novel treatment algorithm based on existing guidelines, the aim of which was to allow all care providers to adequately treat adult AR (Hellings *et al.,* 2020: available from www.euforea.eu). In general, management of AR includes education, allergen avoidance, pharmacotherapy and allergen immunotherapy (AIT). However, the specific management of each patient is influenced by the frequency and intensity of symptoms, response to treatment, the presence of comorbid conditions as well as the patient's age and preference.

As first-line therapy, in patients presenting with two or more nasal symptoms suggestive of AR, the EUFOREA algorithm recommends an intranasal corticosteroid (INCS) and/or an oral non-sedating (anti-H1) antihistamine or an intranasal antihistamine.

As a second-line therapy, in patients whose symptoms are uncontrolled with the first-line therapy, the EUFOREA algorithm recommends treatment be "stepped up" to a fixed dose combination (FDC) of an INCS/intranasal antihistamine, or a combination of INCS and an oral antihistamine.

As a third-line therapy, in patients whose symptoms are uncontrolled with the second-line therapy or in patients who present with so-called "severe" AR (defined in one instance by uncontrolled symptoms despite previous treatment and after confirmation of diagnosis, medication adherence and management of co-morbidities), the EUFOREA algorithm recommends symptom-directed add-on therapies to the second-line, i.e., to the fixed dose combination (FDC) of an INCS/intranasal antihistamine, or a combination of INCS and an oral antihistamine. Symptom directed add-on therapies include: (i) isolated watery rhinorrhea - ipratropium; (ii) rhinorrhea in asthmatics - leukotriene receptor antagonist; (iii) ocular itch/skin rash - oral non-sedating anti-H1 antihistamine; (iv) ocular symptoms - intra-ocular anti-H1 antihistamine or cromones; (v) sudden onset nasal blockage - nasal / oral decongestant for ≤ 7 days. Other third-line therapies (not as add-on therapy) include allergen immunotherapy, short course oral corticosteroids and surgery (for those with severe pharmacological therapy-resistant nasal obstruction). These therapies are typically prescribed at the physician's discretion.

Following the EUFOREA algorithm and in view of the wide range of existing treatment options for AR falling within the scope of the algorithm Wise et al., published the so-called International Consensus Statement on AR (Wise et al., Int Forum Allergy Rhinol, 2023). The consensus statement provided a comprehensive review of the evidence of efficacy for the different drugs used to treat AR, including intranasal and oral antihistamines, intranasal, oral, and injectable corticosteroids, oral and intranasal decongestants, leukotriene receptor antagonist (LTRAs), oral cromolyn, intranasal anticholinergics and biologics (omalizumab) (Wise et al., Table 1).

Consequently, numerous recommendations indicating the suitability of various active ingredients for and against the treatment of AR (in view of variable disease severity and duration) are provided by the consensus statement, which reflects the complexity of treating the condition.

Moreover, whilst there appears to be general agreement between the EUFOREA algorithm and the International Consensus Statement with respect to the recommended first-line therapies of intranasal corticosteroid and/or an oral non-sedating (anti-H1) antihistamine or an intranasal antihistamine (all of which are listed as "Strong recommendation" for treatment by the consensus), there is some disagreement regarding the comparatively more difficult to treat patients, i.e., those whose symptoms are uncontrolled. For example, with respect to the treatment of patients whose symptoms are uncontrolled by first-line therapy, whilst the EUFOREA algorithm recommends symptom-directed add-on therapy of leukotriene receptor antagonists to FDCs of INCS/intranasal antihistamine, or INCS and an oral antihistamine, the consensus statement recommends against the use of leukotriene receptor antagonists in the treatment of allergic rhinitis, except in select situations where patients display contraindications to alternative treatment options. Equally, whilst acknowledging that combinations of oral antihistamines and leukotriene receptor antagonists have been investigated as a first-line therapy for AR, the consensus statement recommends against the use of the combination, except in patients with contraindications to alternative treatment options.

Thus, whilst first-line therapy of AR is well-established, there remains a need in the art to provide further therapeutic options for patients whose symptoms are uncontrolled.

Accordingly, the present invention provides a further therapeutic option for such patients.

### SUMMARY OF THE INVENTION

In a first aspect, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by a single T4NSS measurement evaluation.

In a second aspect, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 48 as calculated by the summation of six T4NSS measurement evaluations.

### DETAILED DESCRIPTION OF THE INVENTION

### Rupatadine

Rupatadine is an antihistamine and has the following structure:

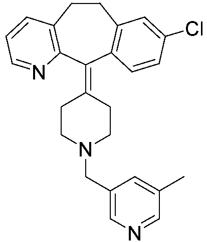

Rupatadine oral tablets (10 mg once daily) are approved in the European Union for the symptomatic treatment of AR (SAR and PAR) and urticaria in adults and adolescents over 12 years of age (Rupafin^{®} - SmPC/PL [ES], 2020). The dosage form is a conventional tablet containing 12.8 mg of rupatadine fumarate (equivalent to 10 mg of rupatadine free base). Specifically, rupatadine is a second-generation antihistamine, it is a long-acting and non-sedating drug that exerts a potent dual-antagonist activity towards the histamine H1 receptor and the PAF receptor.

In the context of the present invention the term "rupatadine" means rupatadine free base or any pharmaceutically acceptable salt thereof. Preferably, it means a pharmaceutically acceptable salt of rupatadine being selected from the group consisting of hydrochloride salt, hydrobromide salt, hydroiodide salt, nitrate salt, perchlorate salt, sulfate salt, phosphate salt, methanesulfonate salt, trifluoromethanesulfonate salt, ethanesulfonate salt, benzenesulfonate salt, p-toluenesulfonate salt, fumarate salt, oxalate salt, maleate salt, citrate salt, succinate salt. More preferably, it means rupatadine fumarate salt.

### Montelukast

Montelukast is a leukotriene receptor antagonist and has the following structure:

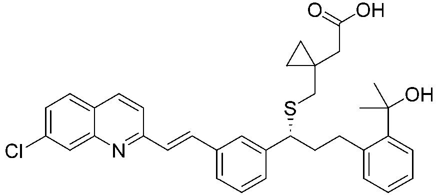

Montelukast film-coated tablets (10 mg once daily) are approved in the European Union in adult and adolescent patients for the treatment of asthma and for symptomatic relief of SAR in asthmatic patients (Singulair^{®} - SmPC/PL [ES], 2023). The dosage form is a conventional tablet containing 10.4 mg of montelukast sodium (equivalent to 10 mg of montelukast free acid). Montelukast is non-sedating and has a safety profile similar in adults and children. Different formulations of montelukast are approved down to 6 months of age, but the tablets are authorised for patients over 15 years of age.

In the context of the present invention the term "montelukast" means montelukast free acid or any pharmaceutically acceptable salt thereof. Preferably, it means a pharmaceutically acceptable salt of montelukast being selected from the group consisting of lithium salt, sodium salt, potassium salt, ammonium salt, calcium salt, magnesium salt, arginine salt, betaine salt, caffeine salt, choline salt, N,N'- dibenzylethylenediamine salt, diethylamine salt, 2-diethylaminoethanol salt, 2-dimethylaminoethanol salt, ethanolamine salt, ethylenediamine salt, N-ethylmorpholine salt, N-ethylpiperidine salt, glucamine salt, glucosamine salt, histidine salt, hydrabamine salt, isopropylamine salt, lysine salt, methylglucamine salt, morpholine salt, piperazine salt, piperidine salt, theobromine salt, triethylamine salt, trimethylamine salt, tripropylamine salt, tromethamine salt, dicyclohexylamine salt. More preferably, it means montelukast sodium salt.

### The pharmaceutical combination of rupatadine and montelukast - the therapeutic uses

As set out above, in a first aspect the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by a single T4NSS measurement evaluation.

In a second aspect, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 48 as calculated by the summation of six T4NSS measurement evaluations.

For the avoidance of any doubt, the determination of a patient's T4NSS is made prior to the patient taking the pharmaceutical combination of the present invention. That is, patients are determined as having a T4NSS of ≥ 8 as calculated by a single T4NSS measurement evaluation, or a T4NSS of ≥ 48 as calculated by the summation of six T4NSS measurement evaluations, prior to treatment.

Despite the consensus statement recommending against the use of oral antihistamines and leukotriene receptor antagonists as a first-line therapy for AR, the inventors rationalised that the combination of rupatadine and montelukast may be particularly suitable for patients suffering with more severe symptoms of SAR, for example, those patients defined by a four-component nasal symptom score (T4NSS) of ≥ 8 as calculated by a single T4NSS measurement evaluation, or those patients defined by a four-component nasal symptom score (T4NSS) ≥ 48 as calculated by the summation of six T4NSS measurement evaluations.

Without wishing to be bound by theory, the inventor's rationale was that the combination of rupatadine and montelukast may provide a suitable and/or improved treatment for said patients based on the additive effect of two different mechanisms of action (mediated by rupatadine and montelukast respectively) to control allergic symptoms. Within the allergic inflammatory cascade, the inventor's considered that the combined effects of the dual-antagonist activity of rupatadine upon the histamine H1 receptor and the PAF receptor, and the blocking effect of montelukast upon type 1 CysLT receptors, may provide an additive effect. That is, taken together, for example as a fixed-dose combination (FDC), the combination may be capable of blocking the most important mediators responsible for the development of the main symptoms associated with SAR pathology (histamine, PAF and leukotriene).

Thus, the inventors concluded that the combination of a second-generation antihistamine like rupatadine and a leukotriene receptor antagonist like montelukast, may be a viable therapeutic option in SAR within the defined patient groups, because successful inhibition of persistent inflammation by the additive effect of two different mechanisms of action may elicit control of more severe symptoms of SAR.

Thus, the pharmaceutical combination of the present invention is for the treatment of seasonal allergic rhinitis (SAR) in patients, wherein the patients are defined by a four-component nasal symptom score (T4NSS).

The T4NSS system is well known in the art. Notably T4NSS or "Total 4 Nasal Symptom Score" is a clinical tool that is frequently used to assess the severity of nasal symptoms in individuals with seasonal allergic rhinitis (SAR).

The T4NSS is a simple, standardised scoring system that evaluates four primary nasal symptoms: (i) nasal congestion - the feeling of blockage or stuffiness in the nasal passages; (ii) rhinorrhea - the amount of nasal discharge or runniness; (iii) sneezing - the frequency of sneezing episodes; and (iv) itchy nose - the level of itching or irritation in the nasal passages. Each symptom is scored on a scale ranging from 0 to 3, where 0 indicates no symptoms and 1, 2, and 3 indicate mild, moderate and severe symptoms respectively. The total score is obtained by adding the individual scores for each of the four symptoms. The higher the score, the more severe the nasal symptoms are, with a maximum score of 12 (if each symptom is rated as 3 and if a measurement of each symptom is recorded once). In a clinical setting, a practitioner may identify a patient's T4NSS by making two T4NSS evaluations and taking the mean score of the two evaluations. Alternatively, a practitioner may identify a patient's T4NSS by making a single T4NSS evaluation.

The skilled person is aware of how to characterise and evaluate the presence of no symptoms, mild symptoms, moderate symptoms and severe symptoms in the context of SAR. For completeness, in the context of the T4NSS evaluation mild symptoms (i.e., score 1) are categorised as clearly present, but with minimal patient awareness - as such, they are easily tolerated. Moderate symptoms (i.e., score 2) are categorised as clearly present with definite patient awareness - as such, they are bothersome. Severe symptoms (i.e., score 3) are categorised as being hard to tolerate to the extent they cause interference with daily activities and/or the ability to sleep. The T4NSS scoring system, and its use, is set out in detail in Ellis et al., Allergy, Asthma & Clinical Immunology, 2015.

Thus, as set out above, in a first aspect, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by a single T4NSS measurement evaluation.

Accordingly, a patient may be identified by performing a single T4NSS measurement evaluation. For example, upon experiencing one or more SAR symptoms, a patient may be seen by a physician who will perform a T4NSS measurement evaluation. If the result of that evaluation is a score of ≥ 8, then the patient falls within the scope of this embodiment.

In one embodiment, a patient may be defined by a four-component nasal symptom score (T4NSS) of ≥ 8 to 12. In one embodiment, a patient may be defined by a four-component nasal symptom score (T4NSS) of ≥ 8 to 12 as determined by a single T4NSS measurement evaluation.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by the mean of two T4NSS measurement evaluations.

In one embodiment, the patient is defined by a four-component nasal symptom score (T4NSS) of ≥ 8 as calculated by the mean of two T4NSS measurement evaluations over one day.

According to this embodiment, measurements are performed twice in one day, for example once in the morning and once in the evening. Typically, measurements may be recorded once at the beginning of a 12-hour period and once at the end of a 12-hour period within a single day. For example, a T4NSS evaluation may be conducted at 8am, which denotes the first daily measurement and the beginning of the 12-hour period, and once at 8pm, which denotes the second daily measurement at the end of the 12-hour period. Measurements recorded once at the beginning of a 12-hour period and once at the end of a 12-hour period within a single day may also be referred to "reflective" measurements.

As also set out above, in a second aspect, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 48 as calculated by the summation of six T4NSS measurement evaluations.

For the avoidance of any doubt, where the four-component nasal symptom score is calculated by the summation of six T4NSS measurement evaluations, the patient's score cannot exceed 72.

Thus, in one embodiment, the invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of from 48 to 72 as calculated by the summation of six T4NSS measurement evaluations.

In one embodiment, the patient is defined by a four-component nasal symptom score (T4NSS) of ≥ 48 as calculated by the summation of six T4NSS measurement evaluations, wherein the measurements are performed over a period of six days.

In one embodiment, the patient is defined by a four-component nasal symptom score (T4NSS) of ≥ 48 as calculated by the summation of six T4NSS measurement evaluations, wherein the measurements are performed once daily over a period of six days.

In one embodiment, the patient is defined by a four-component nasal symptom score (T4NSS) of ≥ 48 as calculated by the summation of six T4NSS measurement evaluations, wherein the measurements are performed over a period of ten days.

In one embodiment, the patient is defined by a four-component nasal symptom score (T4NSS) of ≥ 48 as calculated by the summation of six T4NSS measurement evaluations, wherein the measurements are performed over a period of twelve days.

In one embodiment, the patient is defined by a four-component nasal symptom score (T4NSS) of ≥ 48 as calculated by the summation of six T4NSS measurement evaluations, wherein the measurements are performed over a period of eleven days and measurements are taken every other day. For example, a measurement is taken on day one, no measurement is taken on day 2, a measurement is taken on day three etc.. In a further embodiment, the patient is defined by a four-component nasal symptom score (T4NSS) of ≥ 48 as calculated by the summation of six T4NSS measurement evaluations, wherein the measurements are performed every other day.

In a particular embodiment, the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements.

According to this embodiment, measurements are performed up to twice a day, for example once in the morning and once in the evening as described hereinabove.

Also, according to this embodiment, measurements are performed up to twice a day over a period of four to seven days and include at least two non-consecutive measurements. For the avoidance of any doubt, the inclusion of at least two non-consecutive measurements refers to the requirement that the six T4NSS measurement evaluations cannot be recorded consecutively. To simplify and exemplify the requirement for at least two non-consecutive measurements, the following four-day and six-day measurement scenarios, both of which are representative of the aforementioned embodiment are described.

For the four-day measurement scenario, T4NSS measurement evaluations may be conducted as follows:
Day 1 - AM measure, PM no measure; Day 2 - AM measure, PM measure; Day 3 - AM no measure, PM measure; and Day 4 - AM measure, PM measure.

In this scenario, six T4NSS measurement evaluations, wherein the measurements are performed up to twice a day over a period of four days including at least two non-consecutive measurements have been performed. This is because six measurements have been recorded across a four-day period, including two non-consecutive measurements, specifically Day 1 PM (splitting the Day 1 AM and Day 2 AM measurements) and Day 3 AM (splitting the Day 2 PM and Day 3 PM measurements).

For the six-day measurement scenario, T4NSS evaluations may be measured as follows:
Day 1 - AM measure, PM no measure; Day 2 - AM measure, PM no measure; Day 3 - AM no measure, PM measure; Day 4 - AM no measure, PM no measure; Day 5 - AM measure, PM measure; and Day 6, AM measure.

In this scenario, six T4NSS measurement evaluations, wherein the measurements are performed up to twice a day over a period of six days including at least two non-consecutive measurements have been performed. This is because six measurements have been recorded across a six-day period, including at least two non-consecutive measurements, for example Day 1 PM (splitting the Day 1 AM and Day 2 AM measurements) and Day 3 AM (splitting the Day 2 PM and Day 3 PM measurements).

Other measurement scenarios which meet the requirement that measurements are performed up to twice a day over a period of four to seven days and include at least two non-consecutive measurements, will be readily apparent to the skilled person.

In one embodiment according to the first and second aspects of the invention, the pharmaceutical combination is for use in the treatment of moderate seasonal allergic rhinitis or severe seasonal allergic rhinitis.

The severity of SAR symptoms, and thus the presence of a moderate or severe pathology with respect to SAR, may be assessed and diagnosed by measuring the severity of a patient's symptoms. As discussed hereinabove, the T4NSS measurement evaluations can be used to determine whether a patient is suffering from moderate or severe SAR, with each symptom being scored on a scale ranging from 0 to 3, where 0 indicates no symptoms and 1, 2, and 3 indicate mild, moderate and severe symptoms respectively.

Thus, the pharmaceutical combination of the present invention is directed towards the treatment of patients with moderate or severe SAR, for example, in patients defined by a four-component nasal symptom score (T4NSS) of ≥ 8 as calculated by a single T4NSS measurement evaluation, or in patients defined by a four-component nasal symptom score (T4NSS) of ≥ 48 as calculated by the summation of six T4NSS measurement evaluations.

Furthermore, patients displaying a symptom score provided by 1 x 8 (four symptoms evaluated with score 2, i.e., moderate symptoms); patients displaying a symptom score provided by 1 x 12 (four symptoms evaluated with score 3, i.e., severe symptoms); and those patients in between (defined by symptoms presenting at both the moderate and severe scoring level) may be considered as having moderate to severe SAR by virtue of their symptoms. The same applies equally to patients displaying a symptom score provided by 6 x 8 (four symptoms evaluated with score 2, i.e., moderate symptoms); patients displaying a symptom score provided by 6 x 12 (four symptoms evaluated with score 3, i.e., severe symptoms); and those patients in between (defined by symptoms presenting at both the moderate and severe scoring level).

In theory, patients with moderate or severe SAR may be sub-categorised within the four-component nasal symptom score (T4NSS) of ≥ 8 to 12 as calculated by a single measurement evaluation, or within the four-component nasal symptom score (T4NSS) of ≥ 48 to 72 as calculated by the summation of six T4NSS measurement evaluations.

For example, a patient with moderate SAR may be defined by a four-component nasal symptom score (T4NSS) of from ≥ 8 to < 10 as calculated by a single T4NSS measurement evaluation according to the symptom scoring criteria for moderate/severe symptoms discussed hereinabove. Equally, a patient with severe SAR may be defined by a four-component nasal symptom score (T4NSS) of 11 or 12 as calculated by a single T4NSS measurement evaluation.

In one embodiment, the present invention provides pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 to < 10 as calculated by a single T4NSS measurement evaluation.

In a further embodiment, the present invention provides pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 to < 10 as calculated by the mean of two T4NSS measurement evaluations over a period of one day.

Equally, a patient with moderate SAR may be defined by a four-component nasal symptom score (T4NSS) of from ≥ 48 to < 60 as calculated by the summation of six T4NSS measurement evaluations according to the symptom scoring criteria for moderate/severe symptoms discussed hereinabove. Equally, a patient with severe SAR may be defined by a four-component nasal symptom score (T4NSS) of from ≥ 60 to ≤ 72 as calculated by the summation of six T4NSS measurement evaluations.

In one embodiment, the present invention provides pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 48 to < 60 as calculated by the summation of six T4NSS measurement evaluations. In particular, the patient may be defined by a four-component nasal symptom score (T4NSS) of ≥ 48 to < 57.

In a further embodiment, the present invention provides pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the four-component nasal symptom score (T4NSS) of ≥ 48 to < 60 is calculated by the summation of six T4NSS measurement evaluations, wherein the measurements are performed up to twice a day over a period of four to seven days and include at least two non-consecutive measurements. In particular, the patients may be defined by a four-component nasal symptom score (T4NSS) of ≥ 48 to < 57.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 48 as calculated by the summation of six T4NSS measurement evaluations.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 48 as calculated by the summation of six T4NSS measurement evaluations according to any of the measurement embodiments described hereinabove.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 48, wherein the T4NSS is calculated by the summation of six T4NSS measurement evaluations, wherein the measurements are performed up to twice a day over a period of four to seven days and include at least two non-consecutive measurements.

The skilled person is aware of the existence, pathology and symptoms of bronchial asthma as well as it's severities.

Briefly, it is generally accepted that in mild bronchial asthma, symptoms occur more than twice a week but less than once a day, and flare-ups may affect activity. Furthermore, nighttime flare-ups may occur more often than twice a month but less than once a week. Lung function is 80% of normal or greater in these patients. Equally, in moderate bronchial asthma, symptoms occur daily. Furthermore, flare-ups typically occur and usually last several days. Coughing and wheezing may disrupt normal activities and make it difficult to sleep. Nighttime flare-ups may occur more than once a week. In moderate bronchial asthma, lung function is roughly between 60% and 80% of normal, without treatment. Mild bronchial asthma is typically categorised by "STEPS 1-2" of the GINA (Global Initiative for Asthma) Guidelines, and moderate bronchial asthma is typically categorised by "STEP 3" of the GINA Guidelines (see "Pocket Guide for Asthma Management and Prevention" available from: https://ginasthma.org/wp-content/uploads/2023/07/GINA-2023-Pocket-Guide-WMS.pdf as of March 2025).

Allergic rhinitis and asthma have high comorbidity. Over 80% of asthmatics suffer from allergic rhinitis, while 10-40% of individuals with allergic rhinitis have asthma. Indeed, allergic rhinitis is a risk factor for asthma and the diagnosis of allergic rhinitis can precede a diagnosis of asthma.

Turning to seasonal allergic rhinitis and mild/moderate bronchial asthma, a connection exists between these conditions because bronchial asthma and seasonal allergic rhinitis share allergic mechanisms and triggers, particularly to allergens such as pollen. Consequently, these conditions often coexist, with seasonal allergic rhinitis potentially worsening asthma symptoms. Thus, whilst the pharmaceutical combination of the present invention is primarily directed towards the treatment of SAR in patients, wherein the patients are defined by a four-component nasal symptom score (T4NSS), the combination may also be suitable for indirectly treating or reliving mild/moderate bronchial asthma within said patients. Equally, by virtue of their related/overlapping pathologies, the pharmaceutical combination may also be suitable for directly treating or reliving mild/moderate bronchial asthma within said patients.

In one embodiment, according to the first and second aspects of the invention, the pharmaceutical combination may be suitable for preventing or protecting against a seasonal decrease in lung function, which may present in patients presenting with SAR as defined herein, and mild or moderate bronchial asthma.

In one embodiment, according to the first and second aspects of the invention, the present invention also provides a reduction in the number of asthma attacks in a patient with mild or moderate bronchial asthma and seasonal allergic rhinitis (SAR).

In one embodiment, according to the first and second aspects of the invention, the present invention also provides a reduction in the number of nocturnal awakenings in a patient with mild or moderate bronchial asthma and seasonal allergic rhinitis (SAR).

In a further embodiment, according to the first and second aspects of the invention, the present invention also provides a reduction in the number of asthma attacks and the number of nocturnal awakenings in a patient with mild or moderate bronchial asthma and seasonal allergic rhinitis (SAR).

For the avoidance of any doubt, the reduction in the number of asthma attacks and the number of nocturnal awakenings is a reduction compared to treatment with an antihistamine, such as rupatadine, only.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient without mild or moderate bronchial asthma, wherein the patient is defined by a four-component nasal symptom score (T4NSS) of ≥ 48 as calculated by the summation of six T4NSS measurement evaluations.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient without mild or moderate bronchial asthma, wherein the patient is defined by a four-component nasal symptom score (T4NSS) of ≥ 48 as calculated by the summation of six T4NSS measurement evaluations according to any of the measurement embodiments described hereinabove.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements.

In a further embodiment, present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day and wherein the treatment of SAR comprises the symptomatic treatment of SAR.

In a further embodiment, present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 48 as calculated by the summation of six T4NSS measurement evaluations, and wherein the treatment of SAR comprises the symptomatic treatment of SAR.

That is, the pharmaceutical combination is suitable for treating, lessening or alleviating any of the symptoms associated with SAR in any of the patients defined herein.

In one embodiment, according to the first and second aspects of the invention, the pharmaceutical combination is for use in the symptomatic treatment of seasonal allergic rhinitis, wherein symptomatic treatment is provided by the treatment of one or more symptoms selected from:
- nasal blockade / congestion;
- rhinorrhea (running nose);
- nasal itching;
- sneezing;
- pharyngeal itching;
- tearing; and
- conjunctival redness / ocular pruritus

In one embodiment, according to the first and second aspects of the invention, the pharmaceutical combination is for use in the symptomatic treatment of seasonal allergic rhinitis, wherein symptomatic treatment is provided by the treatment of:
- nasal blockade / congestion, rhinorrhea (running nose, nasal itching; and/or sneezing; and/or
- pharyngeal itching, tearing and/or conjunctival redness / ocular pruritus

In one embodiment, according to the first and second aspects of the invention, the pharmaceutical combination is for use in the symptomatic treatment of seasonal allergic rhinitis, wherein symptomatic treatment is provided by the treatment of sneezing and/or rhinorrhea.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, and wherein the treatment of SAR is symptomatic treatment.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is symptomatic treatment.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations according to any of the measurement embodiments described hereinabove, wherein the treatment of SAR is symptomatic treatment.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, and wherein the treatment of SAR is symptomatic treatment.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, and wherein the treatment of SAR is provided by the treatment of sneezing and/or rhinorrhea.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, and wherein the treatment of SAR is provided by the treatment of sneezing and/or rhinorrhea.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations according to any of the measurement embodiments described hereinabove, and wherein the treatment of SAR is provided by the treatment of sneezing and/or rhinorrhea.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, and wherein the treatment of SAR is provided by the treatment of sneezing and/or rhinorrhea.

In a further embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is symptomatic treatment and wherein the treatment provides a reduction in the number of asthma attacks and/or a reduction in the number of nocturnal awakenings.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 48 as calculated by the summation of six T4NSS measurement evaluations, wherein the treatment of SAR is symptomatic treatment and wherein the treatment provides a reduction in the number of asthma attacks and/or a reduction in the number of nocturnal awakenings.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is provided by the treatment of sneezing and/or rhinorrhea and wherein the treatment provides a reduction in the number of asthma attacks and/or a reduction in the number of nocturnal awakenings

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with mild or moderate bronchial asthma, wherein the patient is defined by a four-component nasal symptom score (T4NSS) of ≥ 48 as calculated by the summation of six T4NSS measurement evaluations, wherein the treatment of SAR is provided by the treatment of sneezing and/or rhinorrhea and wherein the treatment provides a reduction in the number of asthma attacks and/or a reduction in the number of nocturnal awakenings.

For the avoidance of any doubt, the pharmaceutical combination as described by any of the aforementioned embodiments may be suitable for use in the symptomatic treatment of seasonal allergic rhinitis.

Equally, the pharmaceutical combination as described by any of the aforementioned embodiments may be provided as a pharmaceutical composition. For example, the present invention also relates to a pharmaceutical composition comprising rupatadine or a pharmaceutically acceptable salt thereof, montelukast or a pharmaceutically acceptable salt thereof and one or more pharmaceutically acceptable excipients, wherein the composition is for use in the treatment of seasonal allergic rhinitis (SAR) in patients, wherein the patients are defined by a four-component nasal symptom score (T4NSS) as described and defined herein.

The pharmaceutical combination is typically formulated as an oral dosage form adapted for separate, sequential or simultaneous administration.

Consequently, rupatadine or a pharmaceutically acceptable salt thereof and montelukast or a pharmaceutically acceptable salt thereof may be formulated as separate oral dosage forms and administered separately, sequentially or simultaneously. Alternatively, rupatadine or a pharmaceutically acceptable salt thereof and montelukast or a pharmaceutically acceptable salt thereof may be formulated together as a single oral dosage form and administered simultaneously.

For the avoidance of any doubt, separate administration may take place by administering rupatadine, waiting a predetermined amount of time, and then administering montelukast, or vice versa. For example, rupatadine may be administered, and montelukast separately administered five or ten minutes after the rupatadine. Sequential administration may take place by administering rupatadine directly before montelukast, such as successively (without waiting a predetermined amount of time) in two separate dosage forms, or vice versa. Simultaneous administration may take place by administering rupatadine and montelukast together, i.e., in the same mouthful, in two separate dosage forms, or in a single dosage form where the active ingredients are both contained.

In one embodiment, rupatadine and montelukast or pharmaceutically acceptable salts thereof are provided in separate oral dosage forms and administered sequentially or simultaneously. In a further embodiment, rupatadine and montelukast or pharmaceutically acceptable salts thereof are provided as a fixed-dose composition in a single oral dosage form and administered simultaneously.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is symptomatic treatment and wherein the combination is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is symptomatic treatment and wherein the combination is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations according to any of the measurement embodiments described hereinabove, wherein the treatment of SAR is symptomatic treatment and wherein the combination is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the treatment of SAR is symptomatic treatment and wherein the combination is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is provided by the treatment of sneezing and/or rhinorrhea and wherein the combination is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is provided by the treatment of sneezing and/or rhinorrhea and wherein the combination is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations according to any of the measurement embodiments described hereinabove, wherein the treatment of SAR is provided by the treatment of sneezing and/or rhinorrhea and wherein the combination is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the treatment of SAR is provided by the treatment of sneezing and/or rhinorrhea and wherein the combination is administered once daily.

In any of the embodiments mentioned herein, the pharmaceutical combination may be administered once daily for at least 7 days, 14 days, 21 days, 28 days, 35 days, 42 days, 49 days, 56 days, 63 days, 70 days, 77 days or 84 days.

In any of the embodiments mentioned herein, the pharmaceutical combination may be administered once daily for 1 to 7 days; 1 to 14 days; 1 to 21 days; or 1 to 28 days.

In any of the embodiments mentioned herein, the pharmaceutical combination may be administered once daily for up to 7 days, 14 days, 21 days, 28 days, 35 days, 42 days, 49 days, 56 days, 63 days, 70 days, 77 days or 84 days.

In any of the embodiments mentioned herein, the pharmaceutical combination may be administered once daily for at least 1 month, 2 months, 3 months, 4 months, 5 months or 6 months.

In any of the embodiments mentioned herein, the pharmaceutical combination may be administered once daily for up to 1 month, 2 months, 3 months, 4 months, 5 months or 6 months.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, and wherein the combination is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is provided by the treatment of sneezing and/or rhinorrhea, and wherein the combination is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the combination is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is provided by the treatment of sneezing and/or rhinorrhea, and wherein the combination is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations according to any of the measurement embodiments described hereinabove, wherein the treatment of SAR is provided by the treatment of sneezing and/or rhinorrhea, and wherein the combination is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the treatment of is SAR provided by the treatment of sneezing and/or rhinorrhea, and wherein the combination is administered once daily for at least 7 days, 14 days, 21 days or 28 days.

In any of the embodiments mentioned herein, the pharmaceutical combination may provide symptomatic treatment of sneezing and/or rhinorrhea following once-daily administration of the combination for at least 7 days or 14 days. Equally, in any of the embodiments mentioned herein, the pharmaceutical combination may provide symptomatic treatment of sneezing and/or rhinorrhea following once-daily administration of the combination for up to 7 days or 14 days, or for 7 days or 14 days.

In one embodiment, the pharmaceutical combination is suitable for the treatment of patients of from 15 to 18 years of age, 19 to 64 years of age, or ≤ 65 years of age.

As noted hereinabove, the pharmaceutical combination may be formulated as separate oral dosage forms or may be formulated together as a single dosage form, for example as a fixed-dose combination.

In one embodiment, the pharmaceutical combination comprises a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid. The first and second oral dosage forms are separate oral dosage forms, and may be administered to a patient separately, sequentially or simultaneously. For example, simultaneously. In a further embodiment, the pharmaceutical combination comprises a first oral dosage form comprising rupatadine fumarate in an amount of from 11 to 14 mg, and a second oral dosage form comprising montelukast sodium in an amount of from 9 to 11 mg, expressed as weight of montelukast free acid.

In one embodiment, the pharmaceutical combination comprises a single oral dosage form comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid. The single oral dosage form is typically a fixed-dose composition and the rupatadine and montelukast are administered simultaneously (by virtue of a patient ingesting the fixed-dose composition). In a further embodiment, the pharmaceutical combination comprises a single oral dosage form comprising rupatadine fumarate in an amount of from 11 to 14 mg, and montelukast sodium in an amount of from 9 to 11 mg, expressed as weight of montelukast free acid.

Further embodiments relating to dosage regimens are as follows.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the combination is formulated as:
(i) a first oral dosage comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is symptomatic treatment, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is symptomatic treatment, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the treatment of SAR is symptomatic treatment, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage for is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is provided by the treatment of sneezing and/or rhinorrhea, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is provided by the treatment of sneezing and/or rhinorrhea, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the treatment of SAR is provided by the treatment of sneezing and/or rhinorrhea, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, wherein the dosage form is administered once daily.

Typically, the fixed-dose composition comprises rupatadine or a pharmaceutically acceptable salt thereof in an amount of from 11 to 14 mg, expressed as weight of rupatadine free base, and montelukast or a pharmaceutically acceptable salt thereof in an amount of from 9 to 11 mg, expressed as weight of montelukast free acid.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is symptomatic treatment, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is symptomatic treatment, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the treatment of SAR is symptomatic treatment, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 8 as calculated by a single T4NSS measurement evaluation or wherein the patient is defined by a four-component nasal symptom score (T4NSS) of ≥ 8 as calculated by the mean of two T4NSS measurement evaluations over a period of one day, wherein the treatment of SAR is provided by the treatment of sneezing and/or rhinorrhea, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the treatment of SAR is provided by the treatment of sneezing and/or rhinorrhea, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base, and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

In one embodiment, the present invention provides a pharmaceutical combination comprising rupatadine or a pharmaceutically acceptable salt thereof and montelukast or a pharmaceutically acceptable salt thereof for use in the treatment of seasonal allergic rhinitis (SAR) in a patient with or without mild or moderate bronchial asthma, wherein the patient is defined by a four-component nasal symptom score (T4NSS) of ≥ 48 as calculated by the summation of six T4NSS measurement evaluations, wherein the measurements are performed up to twice a day over a period of four to seven days and include at least two non-consecutive measurements, wherein the treatment of SAR is provided by the treatment of sneezing and/or rhinorrhea, wherein the combination is formulated as:
(i) a first oral dosage form comprising rupatadine or a pharmaceutically acceptable salt thereof in an amount of from 0.5 to 22 mg, expressed as weight of rupatadine free base, and a second oral dosage form comprising montelukast or a pharmaceutically acceptable salt thereof in an amount of from 1 to 22 mg, expressed as weight of montelukast free acid, and wherein the first and second dosage forms are administered sequentially or simultaneously, once daily, for at least 7 days, 14 days, 21 days or 28 days; or
(ii) a single oral dosage form fixed-dose composition comprising rupatadine or a pharmaceutically acceptable salt thereof in an amount of from 0.5 to 22 mg, expressed as weight of rupatadine free base,and montelukast or a pharmaceutically acceptable salt thereof in an amount of from 1 to 22 mg, expressed as weight of montelukast free acid, and wherein the dosage form is administered once daily for at least 7 days, 14 days, 21 days or 28 days.

Typically, the fixed-dose composition is a tablet composition, for example a monolayer or bilayer tablet composition.

Typically, the fixed-dose composition comprises rupatadine fumarate in an amount of from 11 to 14 mg, and montelukast sodium in an amount of from 9 to 11 mg.

In one embodiment, the pharmaceutical combination comprises rupatadine or a pharmaceutically acceptable salt thereof in an amount of about 10 mg expressed as rupatadine free base, and montelukast or a pharmaceutically acceptable salt thereof in an amount of about 10 mg expressed as montelukast free acid.

In a particular embodiment, the pharmaceutical combination is a fixed-dose composition comprising rupatadine or a pharmaceutically acceptable salt thereof in an amount of about 10 mg expressed as rupatadine free base, and montelukast or a pharmaceutically acceptable salt thereof in an amount of about 10 mg expressed as montelukast free acid.

In many of the embodiments disclosed herein, treatment is specified as being directed to a patient without mild or moderate asthma. For the avoidance of any doubt, the patients of these embodiments are patients not only without mild or moderate bronchial asthma, but they are also without any severity of bronchial asthma. That is, they do not have asthma.

In many of the embodiments disclosed herein, treatment is specified as being directed to a patient with mild or moderate asthma. In all these embodiments, it is preferable that the patients have mild asthma.

In other aspects, the invention provides:
A pharmaceutical combination comprising rupatadine or a pharmaceutically acceptable salt thereof and montelukast or a pharmaceutically acceptable salt thereof for the manufacture of a medicament for the treatment of seasonal allergic rhinitis (SAR) in a patient, wherein the patient is defined by a four-component nasal symptom score (T4NSS) of ≥ 8 as calculated by a single T4NSS measurement evaluation.

A method for the treatment of seasonal allergic rhinitis (SAR), which comprises administering to a patient or a subject in need thereof a pharmaceutical combination comprising rupatadine or a pharmaceutically acceptable salt thereof and montelukast or a pharmaceutically acceptable salt thereof, wherein the patients are defined by a four-component nasal symptom score (T4NSS) of ≥ 8 as calculated by a single T4NSS measurement evaluation.

A pharmaceutical combination comprising rupatadine or a pharmaceutically acceptable salt thereof and montelukast or a pharmaceutically acceptable salt thereof for the manufacture of a medicament for the treatment of seasonal allergic rhinitis (SAR) in a patient, wherein the patient is defined by a four-component nasal symptom score (T4NSS) of ≥ 48 as calculated by the summation of six T4NSS measurement evaluations.

A method for the treatment of seasonal allergic rhinitis (SAR), which comprises administering to a patient or subject in need thereof a pharmaceutical combination comprising rupatadine or a pharmaceutically acceptable salt thereof and montelukast or a pharmaceutically acceptable salt thereof, wherein the patient is defined by a four-component nasal symptom score (T4NSS) of ≥ 48 as calculated by the summation of six T4NSS measurement evaluations.

For the avoidance of any doubt, the manufacture of a medicament and the method of treatment aspects also encompass all the "for use" treatment aspects and embodiments disclosed herein. Furthermore, in all aspects and embodiments of the present invention, patients may be considered as subjects or indeed subjects in need of therapy, i.e,, subjects in need thereof.

Further embodiments of the invention may also include:
A pharmaceutical combination comprising rupatadine or a pharmaceutically acceptable salt thereof and montelukast or a pharmaceutically acceptable salt thereof for use in the treatment of moderate seasonal allergic rhinitis (SAR).

A pharmaceutical combination comprising rupatadine or a pharmaceutically acceptable salt thereof and montelukast or a pharmaceutically acceptable salt thereof for use in the treatment of seasonal allergic rhinitis (SAR) in a patient or subject defined as having moderate SAR according to a T4NSS measurement evaluation.

A pharmaceutical combination comprising rupatadine or a pharmaceutically acceptable salt thereof and montelukast or a pharmaceutically acceptable salt thereof for use in the treatment of moderate seasonal allergic rhinitis (SAR) in a patient with or without mild bronchial asthma.

A pharmaceutical combination comprising rupatadine or a pharmaceutically acceptable salt thereof and montelukast or a pharmaceutically acceptable salt thereof for use in the treatment of moderate seasonal allergic rhinitis (SAR) in a patient without bronchial asthma.

A pharmaceutical combination comprising rupatadine or a pharmaceutically acceptable salt thereof and montelukast or a pharmaceutically acceptable salt thereof for use in the treatment of seasonal allergic rhinitis (SAR) in a patient or subject defined as having moderate SAR according to a T4NSS measurement evaluation in a patient with or without mild bronchial asthma.

A pharmaceutical combination comprising rupatadine or a pharmaceutically acceptable salt thereof and montelukast or a pharmaceutically acceptable salt thereof for use in the treatment of seasonal allergic rhinitis (SAR) in a patient or subject defined as having moderate SAR according to a T4NSS measurement evaluation in a patient without mild bronchial asthma.

A pharmaceutical combination comprising rupatadine or a pharmaceutically acceptable salt thereof and montelukast or a pharmaceutically acceptable salt thereof for use in the treatment of severe seasonal allergic rhinitis (SAR).

A pharmaceutical combination comprising rupatadine or a pharmaceutically acceptable salt thereof and montelukast or a pharmaceutically acceptable salt thereof for use in the treatment of seasonal allergic rhinitis (SAR) in a patient or subject defined as having severe SAR according to a T4NSS measurement evaluation.

A pharmaceutical combination comprising rupatadine or a pharmaceutically acceptable salt thereof and montelukast or a pharmaceutically acceptable salt thereof for use in the treatment of severe seasonal allergic rhinitis (SAR) in a patient with or without mild bronchial asthma.

A pharmaceutical combination comprising rupatadine or a pharmaceutically acceptable salt thereof and montelukast or a pharmaceutically acceptable salt thereof for use in the treatment of severe seasonal allergic rhinitis (SAR) in a patient without bronchial asthma.

A pharmaceutical combination comprising rupatadine or a pharmaceutically acceptable salt thereof and montelukast or a pharmaceutically acceptable salt thereof for use in the treatment of seasonal allergic rhinitis (SAR) in a patient or subject defined as having severe SAR according to a T4NSS measurement evaluation in a patient with or without mild bronchial asthma.

A pharmaceutical combination comprising rupatadine or a pharmaceutically acceptable salt thereof and montelukast or a pharmaceutically acceptable salt thereof for use in the treatment of seasonal allergic rhinitis (SAR) in a patient or subject defined as having severe SAR according to a T4NSS measurement evaluation in a patient without bronchial asthma.

### The pharmaceutical combination of rupatadine and montelukast - the composition

As noted hereinabove, the present invention also relates to a pharmaceutical composition comprising rupatadine or a pharmaceutically acceptable salt thereof, montelukast or a pharmaceutically acceptable salt thereof and one or more pharmaceutically acceptable excipients.

That is, the pharmaceutical combination according to any of the embodiments as described herein, may typically be formulated as a pharmaceutical composition, for example as a composition comprising rupatadine, montelukast and one or more pharmaceutically acceptable excipients, for example as a fixed-dose composition.

The pharmaceutically acceptable excipient(s) can be selected from, for example, carriers (e.g. a solid, liquid or semi-solid carrier), adjuvants, diluents, fillers or bulking agents, granulating agents, coating agents, release-controlling agents, binding agents, disintegrants, lubricating agents, preservatives, antioxidants, buffering agents, suspending agents, thickening agents, flavouring agents, sweeteners, taste masking agents, stabilisers, anti-caking agents, flow agents or any other excipients conventionally used in pharmaceutical compositions.

The term "pharmaceutically acceptable" as used herein pertains to compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation. Thus, to the extent an excipient is pharmaceutically acceptable it can also be considered nutraceutically acceptable i.e., an excipient that is an inactive substance used in the formulation of nutraceutical products, foods or dietary supplements which provide health benefits beyond basic nutrition.

The pharmaceutical composition of the present invention may be formulated in accordance with known techniques, see for example, Remington's Pharmaceutical Sciences, Mack Publishing Company, Easton, PA, USA.

In one embodiment, the composition is formulated as an oral dosage form, that is, as a dosage form suitable for oral administration. Dosage forms suitable for oral administration include tablets (coated or uncoated), capsules (hard or soft shell), caplets, gummies, pills, lozenges, syrups, solutions, powders, granules, elixirs and suspensions, sublingual tablets, wafers or buccal patches.

In one embodiment, the composition is formulated as an oral dosage form, wherein the oral dosage is a tablet or a capsule.

In one embodiment, the composition is formulated as an oral dosage form, wherein the oral dosage is a tablet, for example a monolayer or multilayer tablet, for example a bilayer tablet.

Tablet compositions typically contain a unit dosage of rupatadine or a pharmaceutically acceptable salt thereof, montelukast or a pharmaceutically acceptable salt thereof and an inert diluent or carrier such as a sugar or sugar alcohol, e.g. lactose, sucrose, sorbitol or mannitol; and/or a non-sugar derived diluent such as sodium carbonate, calcium phosphate, dicalcium phosphate, calcium carbonate, or a cellulose or derivative thereof such as microcrystalline cellulose (MCC), methyl cellulose, ethyl cellulose, hydroxypropyl methyl cellulose, croscarmellose sodium, hypromellose, glycerin and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), magnesium stearate and silicon dioxide and effervescent agents such as citrate/bicarbonate mixtures. Such excipients are well known and do not need to be discussed in detail here.

Tablets may be designed to release their ingredients immediately or to release their ingredients in a controlled manner (controlled release tablets) over a prolonged period of time or with a specific region of the GI tract. For example, tablets may be designed to release rupatadine and montelukast and one or more pharmaceutically acceptable excipients either upon contact with stomach fluids (immediate release tablets) or to release in a controlled manner (controlled release tablets) over a prolonged period of time or with a specific region of the GI tract.

Capsule formulations may be of the hard gelatin or soft gelatin variety and can contain rupatadine or a pharmaceutically acceptable salt thereof, montelukast or a pharmaceutically acceptable salt thereof, and one or more pharmaceutically acceptable excipients in solid i.e. powder or granule, semi-solid, or liquid form. Gelatin capsules can be formed from animal gelatin or synthetic or plant derived equivalents thereof.

The solid dosage forms (e.g., tablets and capsules) can be coated or uncoated. Coatings may act either as a protective film (e.g. a polymer, wax or varnish) or as a mechanism for controlling release of rupatadine, montelukast and/or the further components of the composition or for aesthetic or identification purposes. The coating (e.g. a Eudragit ^{™} type polymer) can be designed to release the rupatadine, montelukast and/or further components of the respective compositions at a desired location within the gastrointestinal tract. Thus, the coating can be selected to degrade under certain pH conditions within the gastrointestinal tract, thereby selectively release the compound in the stomach or in the ileum, duodenum, jejenum or colon.

The skilled person possesses the expertise to formulate rupatadine and montelukast with pharmaceutically acceptable excipients and to prepare an oral dosage form, for example a monolayer tablet, a multilayer tablet or a capsule.

The pharmaceutically acceptable excipient(s) can be selected according to the desired physical form of the formulation and can, for example, be selected from diluents (e.g solid diluents such as fillers or bulking agents; and liquid diluents such as solvents and co-solvents), disintegrants, buffering agents, lubricants, flow aids, release controlling (e.g. release retarding or delaying polymers or waxes) agents, binders, granulating agents, pigments, plasticizers, antioxidants, preservatives, flavouring agents, taste masking agents, tonicity adjusting agents and coating agents.

In one embodiment, the pharmaceutical composition is formulated to comprise one or more excipients selected from the group consisting of binders, diluents, disintegrants, lubricants, glidants, antioxidants, colorants and flavours.

Disintegrants may aid dispersion of the tablet in the gastrointestinal tract, releasing the active ingredient and increasing the surface area for dissolution. According to one embodiment, disintegrants may be selected from the group comprising alginates such as calcium and/or sodium alginate, calcium carboxymethylcellulose, calcium cellulose glycolate, calcium silicate, carboxy methyl cellulose calcium, starch, croscarmellose sodium, crospovidone, sodium docusate, hydroxypropyl methyl cellulose, hydroxypropyl starch, magnesium aluminum silicate, methylcellulose, polyvinylpyrrolidone, powdered cellulose, sodium carboxy methyl cellulose, sodium starch glycolate, and mixtures thereof. In an embodiment, the disintegrant preferably is sodium croscarmellose. Binders help binding the tablet ingredients together giving form and mechanical strength. According to one embodiment, binders may be selected from the group consisting of acacia mucilage, agar, alginates (sodium), carboxymethylcellulose (sodium or calcium), carbomer, carrageenan, chitosan, copovidone, starch (corn), ethylcellulose, gelatin, hydroxyethyl cellulose, hydroxyethylmethyl cellulose, hydroxypropyl cellulose, hydroxypropyl methyl cellulose, hydroxypropyl starch, magnesium aluminum silicate, methyl cellulose, natural gums (agar, guam, tragachanti), pectin, polyethylene glycols, polyvinylpyrrolidone, pregelatinized starch, and mixtures thereof. In an embodiment, the binders preferably are pregelatinized starch and hydroxypropyl cellulose.

Diluents (also known as fillers) provide bulk and enable accurate dosing of active ingredients. According to one embodiment, binders may be selected from the group consisting of alpha-lactalbumin, anhydrous lactose, calcium carbonate, calcium lactate, calcium phosphate (dibasic, anhydrous and dibasic, dehydrate), calcium sulfate, cellulose acetate, compressible sugar, dextrates, dextrin, dextrose, erythritol, ethyl acrylate and methyl methacrylate copolymer, ethylcellulose, fructose, hydroxypropyl pea starch, isomalt, kaolin, lactitol, lactose monohydrate, magnesium carbonate, magnesium oxide, maize starch, maltodextrin, maltose, mannitol, methacrylic acid and ethyl acrylate copolymer, methacrylic acid and methyl methacrylate copolymer, microcrystalline cellulose, polydextrose, powdered cellulose, pregelatinized hydroxypropyl potato starch, pregelatinized modified starch, starch (corn, potato, maize, pea, tapioca, wheat), pullulan, silicified microcrystalline cellulose, simethicone, sodium bicarbonate, sodium carbonate, sodium chloride, sorbitol, spray-dried lactose, hydrogenated starch hydrolysate, sucrose, trehalose, tribasic calcium phosphate, xylitol and mixtures thereof. In an embodiment, the diluents preferably are lactose monohydrate and microcrystalline cellulose.

Lubricants (also known as glidants) improve the flow of powders during tablet manufacturing by reducing friction and adhesion between particles. According to one embodiment, lubricants may be selected from the group consisting of behenoyl polyoxylglycerides, calcium silicate, calcium stearate, cellulose, powdered colloidal silicon dioxide, fumaric acid, glyceryl behenate, glyceryl dibehenate, glyceryl monostearate, glyceryl palmitostearate, glyceryl tristearate, hydrogenated natural oils, hydrophobic colloidal silica, lauric acid, magnesium lauryl sulphate, magnesium lauryl sulphate, magnesium oxide, magnesium silicate, magnesium stearate, myristic acid, palmitic acid, paraffin, poloxamer, polyethylene glycol, polyethylene glycol, polyethylene glycol, polyoxyl 10 oleyl ether, polyoxyl 15 hydroxystearate, polyoxyl 20 cetostearyl ether, polyoxyl 40 stearate, polysorbates (20, 40, 60, 80), potassium benzoate, silica, sodium benzoate, sodium lauryl sulphate, sodium stearate, sodium stearyl fumarate, sorbitan monolaurate, sorbitan monooleate, sorbitan monopalmitate, sorbitan monostearate, sorbitan sesquioleate, sorbitan trioleate, stearic acid, talc, tribasic calcium phosphate, zinc stearate and mixtures thereof. In an embodiment, the lubricant preferably is magnesium stearate.

In one embodiment the pharmaceutical composition of the invention has a total amount of diluents comprised between 20 wt% and 90 wt% w/w, preferably between 35 wt% and 90 wt%, more preferably between 50 wt% and 90 wt% and more preferably between 70 wt% and 90 wt%, a total amount of binders comprised between 2 and 14 wt%, preferably between 2 wt% and 11 wt%, more preferably between 3 and 9% and more preferably between 4 wt% and 7 wt%, a total amount of disintegrants comprised between 1 and 15 wt %, preferably between 1 wt% and 13 wt%, more preferably between 1 and 11 % and more preferably between 1 wt% and 8 wt% and a total amount of lubricants comprised between 0.2 and 2.0 wt%, preferably between 0.3 wt% and 1.7 wt%, more preferably between 0.5 and 1.5% and more preferably between 0.7 wt% and 1.3 wt% all percentages being expressed relative to the total weight of the pharmaceutical combination.

In one embodiment the pharmaceutical composition of the invention comprises as pharmaceutical excipients, pregelatinized starch, microcrystalline cellulose, lactose monohydrate, hydroxypropyl cellulose, sodium croscarmellose and magnesium stearate.

In one embodiment the pharmaceutical composition of the invention has a total amount of pregelatinized starch comprised between 1 wt% and 10 wt% w/w, preferably between 1 .5 wt% and 8 wt%, more preferably between 2 wt% and 6 wt% and more preferably between 2.5 wt% and 4 wt%, a total amount of microcrystalline cellulose comprised between 10 wt% and 60 wt% w/w, preferably between 18 wt% and 52 wt%, more preferably between 25 wt% and 45 wt% and more preferably between 32 wt% and 38 wt%, a total amount of lactose monohydrate comprised between 15 wt% and 75 wt% w/w, preferably between 23 wt% and 68 wt%, more preferably between 30 wt% and 60 wt% and more preferably between 35 wt% and 55 wt%, a total amount of hydroxypropyl cellulose comprised between 0.5 wt% and 8 wt% w/w, preferably between 0.8 wt% and 8 wt%, more preferably between 1 wt% and 6 wt% and more preferably between 1.5 wt% and 3 wt%, a total amount of sodium croscarmellose comprised between 0.5 wt% and 8 wt% w/w, preferably between 0.8 wt% and 8 wt%, more preferably between 1 wt% and 6 wt% and more preferably between 1.5 wt% and 3 wt% and a total amount of magnesium stearate comprised between 0.1 wt% and 2 wt% w/w, preferably between 0.3 wt% and 1.6 wt%, more preferably between 0.5 wt% and 1.4 wt% and more preferably between 0.8 wt% and 1 .2 wt%,

In one embodiment the pharmaceutical composition of the invention has a total amount of diluents comprised between 20 wt% and 90 wt% w/w, preferably between 35 wt% and 90 wt%, more preferably between 50 wt% and 90 wt% and more preferably between 70 wt% and 90 wt%, a total amount of binders comprised between 2 and 14 wt%, preferably between 2 wt% and 11 wt%, more preferably between 3 and 9% and more preferably between 4 wt% and 7 wt%, a total amount of disintegrants comprised between 1 and 15 wt %, preferably between 1 wt% and 13 wt%, more preferably between 1 and 11 % and more preferably between 1 wt% and 8 wt% and a total amount of lubricants comprised between 0.2 and 2.0 wt%, preferably between 0.3 wt% and 1.7 wt%, more preferably between 0.5 and 1.5% and more preferably between 0.7 wt% and 1.3 wt% all percentages being expressed relative to the total weight of the pharmaceutical composition.

In one embodiment, the pharmaceutical composition is a fixed-dose composition, for example a tablet, such as a monolayer or multilayer tablet, comprising a diluent, binder, disintegrant, lubricant, glidant, antioxidant, colourant and/or flavouring agent.

In one embodiment the pharmaceutical composition is a fixed-dose composition, for example a tablet, such as a monolayer or multilayer tablet, comprising a diluent, binder, disintegrant and lubricant, wherein the total amount of diluents is of from 20 wt% and 90 wt% w/w, the total amount of binders is of from 2 and 14 wt%, the total amount of disintegrants is of from 1 and 15 wt % and the total amount of lubricants is of from 0.2 and 2.0 wt%.

In one embodiment the pharmaceutical composition of the invention comprises as pharmaceutical excipients, pregelatinized starch, microcrystalline cellulose, lactose monohydrate, hydroxypropyl cellulose, sodium croscarmellose and magnesium stearate.

In one embodiment the pharmaceutical combination of the invention has a total amount of pregelatinized starch comprised between 1 wt% and 10 wt% w/w, preferably between 1 .5 wt% and 8 wt%, more preferably between 2 wt% and 6 wt% and more preferably between 2.5 wt% and 4 wt%, a total amount of microcrystalline cellulose comprised between 10 wt% and 60 wt% w/w, preferably between 18 wt% and 52 wt%, more preferably between 25 wt% and 45 wt% and more preferably between 32 wt% and 38 wt%, a total amount of lactose monohydrate comprised between 15 wt% and 75 wt% w/w, preferably between 23 wt% and 68 wt%, more preferably between 30 wt% and 60 wt% and more preferably between 35 wt% and 55 wt%, a total amount of hydroxypropyl cellulose comprised between 0.5 wt% and 8 wt% w/w, preferably between 0.8 wt% and 8 wt%, more preferably between 1 wt% and 6 wt% and more preferably between 1.5 wt% and 3 wt%, a total amount of sodium croscarmellose comprised between 0.5 wt% and 8 wt% w/w, preferably between 0.8 wt% and 8 wt%, more preferably between 1 wt% and 6 wt% and more preferably between 1.5 wt% and 3 wt% and a total amount of magnesium stearate comprised between 0.1 wt% and 2 wt% w/w, preferably between 0.3 wt% and 1.6 wt%, more preferably between 0.5 wt% and 1.4 wt% and more preferably between 0.8 wt% and 1.2 wt%,

As noted above, the skilled person possesses the expertise to formulate rupatadine and montelukast with pharmaceutically acceptable excipients and to prepare an oral dosage form, for example a monolayer or multilayer tablet or a capsule.

For example, the skilled person could prepare a bilayer tablet comprising rupatadine and montelukast, as disclosed in WO 2017/182641, or the skilled person could prepare an oral dosage form in which rupatadine and montelukast by granulating rupatadine and montelukast together with pharmaceutically acceptable excipients, as disclosed in WO 2017/182644.

Alternatively, the skilled person could prepare a single layer tablet comprising rupatadine and montelukast, as disclosed in WO2024/126772.

The pharmaceutical combination may be presented to a patient or subject in "patient packs" containing an entire course of treatment in a single package, for example a bottle or blister pack containing tablets or capsules.

### EXAMPLES

The present invention will now be illustrated, but not limited, by reference to the specific embodiments described in the following examples.

### Example 1- Tablet composition and method of preparation

300 mg tablets were prepared with the composition showed in Table A following the process described below:

**Table A:**

| **#** | **Ingredient** | **%** | **Weight/tablet (mg)** |
|---|---|---|---|
| 1 | Rupatadine fumarate (as rupatadine free base) | 4.27 | 12.8 (10.0) |
| 2 | Pregelatinized starch | 3.33 | 10.0 |
| 3 | Microcrystalline cellulose | 5.0 | 15.0 |
| 4 | Lactose monohydrate | 20.4 | 61.2 |
| 5 | Montelukast sodium (as Montelukast free acid) | 3.47 | 10.4 (10) |
| 6 | Lactose monohydrate | 29.33 | 88.0 |
| 7 | Microcrystalline cellulose | 29.29 | 87.87 |
| 8 | Hydroxypropyl cellulose | 1.91 | 5.73 |
| 9 | Sodium croscarmellose | 2.0 | 6.0 |
| 10 | Magnesium stearate | 1.0 | 3.0 |
| | TOTAL | 100.0 | 300.0 |

### Step 1: Preparation of rupatadine fumarate granulate

128 g of rupatadine fumarate, 100 g of pregelatinized starch, 150 g of microcrystalline cellulose and 612 g of lactose monohydrate were blended to prepare a homogeneous mixture. The mixture was then granulated with 216.81 g of water in a high-shear mixer (Mixer-granulator Lleal TRI-CHOP MGR-5/1). The resulting wet granulate was dried in an air-forced oven at a temperature of 45°C.

### Step 2: Preparation of intermediate mixture

768.86 g of microcrystalline cellulose, 770.0 g of lactose monohydrate, 91 g of sodium montelukast and 50.14 g of hydroxypropyl cellulose were blended to prepare a homogeneous mixture.

### Step 3: Preparation of product for compression

52.5 g of sodium croscarmellose were added to 866.25 g of the rupatadine fumarate granules prepared in step 1 followed by 1680.0 g of intermediate mixture prepared in step 2 and the mixture was blended to obtain a homogeneous mixture. Finally, 26.25 mg of magnesium stearate were added to the previous mixture and the mixture was blended to obtain a homogeneous mixture.

### Step 4: Preparation of tablets

The final mixture of step 3 was compressed to a 300.0 mg tablets in a rotary tablet press equipped with round punches having a diameter of 9 mm at a pressure of 4-5 KN.

### Example 2 - Clinical study report: A Randomised, Double-Blind, Two-Arm, Parallel Groups, Multicentre Study to Evaluate the Efficacy and Safety of a Combination of Rupatadine and Montelukast vs. Rupatadine in Adult Patients with Seasonal Allergic Rhinitis with and without Mild or Moderate Bronchial Asthma

### 1 INVESTIGATORS AND STUDY ADMINISTRATIVE STRUCTURE

### 1.1 Investigators

This clinical trial was conducted at 6 sites in Poland, 11 sites in Spain, and 4 sites in Bulgaria. Independent ethics committees (IECs) approved 22 Principal Investigators (PIs) and sites to participate in the trial. Nineteen (19) sites were opened and enrolled in at least one subject. Each country had a national coordinator.

### 2 ETHICS

### 2.1 Ethical Conduct of the Study

This trial was conducted in compliance with the CSP, the International Council for Harmonisation of Technical Requirements for Pharmaceuticals for Human Use (ICH) Good Clinical Practice (GCP) Guideline, the Declaration of Helsinki, applicable national and European laws, directives, and regulations.

### 3 OBJECTIVES AND ENDPOINTS

### 3.1 Study Objective

The objective of this trial was to demonstrate the therapeutic superiority of the test combination product (rupatadine and montelukast) over rupatadine monotherapy in moderate to severe seasonal allergic rhinitis (SAR) patients with and without mild or moderate bronchial asthma inadequately controlled.

### 3.1.1 Primary Endpoint

The main efficacy endpoint was the change from baseline in the mean total four nasal symptom score (T4NSS) over the treatment period (reflective of 12 hours of symptoms, every day including the visit days with the investigator). Baseline score was calculated as the mean of the score provided by the investigator at screening (screening nasal severity score).

### 3.1.2 Secondary Endpoints

- Change from baseline in the mean total three non-nasal symptom score (T3NNSS) over the treatment period (reflective evaluation)
- Change from baseline in the mean total seven symptom score (T7SS) over the treatment period (reflective evaluation)
- Change from baseline in the mean daily symptom score (DSS) for each symptom over the treatment period (reflective evaluation)
- Onset of action for T4NSS, T3NNSS, T7SS, and individual symptoms (DSS)
- Percentage of patients requiring rescue medication

### 3.1.3 Exploratory Endpoints

- Change from baseline in the mean total number of asthma attacks over the treatment period
- Change from baseline in the mean of nocturnal awakenings over the treatment period
- Medication use for asthma

### 3.1.4 Safety Endpoints

- Adverse events (AEs) incidence
- Related adverse events incidence
- Serious adverse events (SAEs) incidence
- Clinically relevant changes in vital signs
- Clinically relevant changes in electrocardiogram (ECG)
- Clinically relevant changes in laboratory results

### 4 INVESTIGATIONAL PLAN

### 4.1 Description of Study Design

This was a randomised, double-blind, two-arm, parallel groups, multicentre phase II proof of concept study to evaluate the efficacy (therapeutic superiority) and safety of the test combination of rupatadine 10 mg and montelukast 10 mg over rupatadine 10 mg monotherapy in adult patients with moderate to severe SAR and with or without mild or moderate bronchial asthma inadequately controlled.

Approximately 360 patients with SAR were planned to be screened to have 300 randomised and at least 240 evaluable subjects. A total of 335 adult patients with SAR were screened, and 301 subjects were randomly assigned to one of the two treatments.

The study duration contained up to a 28-day wash-out run-in period (if required), 4 to 7 days for assessing severity and confirming eligibility, and a 28-day (± 2 days) treatment period. Subjects underwent up to 7 study visits: 2 visits were conducted during the screening period (these visits could be merged under several conditions), 1 visit on the randomisation day, and 4 visits (including the end of treatment [EoT] visit) after starting the treatment.

Eligible subjects were randomly assigned in a 1:1 ratio to a test product treatment group (10 mg tablet of rupatadine and 10 mg tablet of montelukast) and a reference product treatment group (10 mg tablet of rupatadine and 1 placebo tablet). Both rupatadine (Rupafin^{®}) and montelukast (Singulair^{®}) are authorised and on the European market. The IMPs (investigational medicinal product) were taken for 28 ± 2 days (i.e. for the treatment period) once daily orally before the meal in the evening.

### 4.2 Selection of Study Population

The study population consisted of adults with moderate to severe SAR and with or without mild or moderate bronchial asthma. Patients had to meet all the inclusion criteria and none of the exclusion criteria to be enrolled in the trial. The inclusion and exclusion criteria were reviewed at screening and at the baseline visit.

### 4.2.1 Inclusion Criteria

Patients meeting the following criteria were considered for inclusion in the trial:
1. Written informed consent in accordance with applicable regulatory requirements.
2. Male and female outpatients aged 18 or more with at least 1-year history of SAR inadequately controlled and in need of alternative treatments as per the treating physicians with or without mild or moderate allergic bronchial asthma.
3. Female patients of childbearing potential and fertile male patients who were sexually active with a female of childbearing potential had to use methods of contraception (barrier methods and oral contraceptives) throughout the study visits.
4. Positive skin prick test (wheal ± 3 mm larger than the diluent control) to at least one seasonal allergen specific to their geographical location (including a variety of common grass pollens [bermuda, rye, sweet grass, blue grass, perennial rye, and timothy grass] and tree pollens [birch, Cupressus spp., oak, olive, Platanus spp., beech, alder, hazelnut, white ash and Salix spp.]).
5. Evaluation of four nasal symptom score (T4NSS) measured as a reflective way (12 hours apart in morning and evening periods) at screening with a total score of ≥ 48 (out of 72) as the sum of six T4NSS measurements with at least two non-consecutive measurements over a period of four to seven days.
6. Adequate wash-out to baseline if patients were taking any of the medications mentioned in Table 1 below.

**Table 1: Wash-out**

| **Medication** | **Wash-out Period** |
|---|---|
| Systemic corticosteroids | 28 days |
| Ketotifen | 14 days |
| Macrolides | 7 days |
| Imidazole antifungals | 7 days |
| Anticholinergics | 7 days |
| Montelukast | 7 days |
| Cetirizine | 3 days |
| Bilastine | 3 days |
| Levocetirizine | 2 days |
| Loratadine, Desloratadine and Rupatadine or any other oral H1 antihistaminic not listed above | 6 days |
| Topical antihistaminic (nasal or drops) | 2 days |
| Any drug with antihistaminic activity (e.g., cold remedies), such as phenothiazine or derivatives | 6 days |
| Intranasal/systemic decongestants or eye drops | 3 days |

Additional Inclusion Criteria for Patients with Asthma:
1. History of asthma for at least 6 months before screening.
2. Adult patients with mild or moderate asthma partially controlled by beclomethasone dipropionate ≤ 500 µg/d or equivalent according to the Global Initiative for Asthma (GINA) guidelines as shown in the Table 2 below (alone or in combination with long-term β2-adrenergic bronchodilators).

**Table 2:**

| **Medication** | **Dose (µg/day)** |
|---|---|
| Budesonide | ≤ 400 |
| Ciclesonide | ≤ 160 |
| Fluticasone | ≤ 250 |
| Mometasone | ≤ 400 |

### 4.2.2 Exclusion Criteria

Patients who met any of the following criteria were not eligible for the trial:
1. Active acute or chronic pulmonary disorder, acute sinus disease that had not resolved within 1 week (active hay fever and AR symptoms are allowed), and upper respiratory tract infection within 3 weeks, emergency department treatment of asthma within 1 month, and hospitalisation for asthma within 3 months before the randomisation visit.
2. Bronchial asthma patients who received treatment with inhaled corticosteroids (ICS), alone or in combination with short and/or long-term β2-adrenergic bronchodilators, at dosage > 500 µg beclomethasone dipropionate or equivalent (budesonide > 400 µg/d, ciclesonide >160 µg/d, fluticasone >250 µg/d and mometasone >400 µg/d) within 2 weeks.
3. Daily treatment with montelukast within 7 days prior to randomisation, cetirizine and bilastine (3 days), levocetirizine (2 days), loratadine, desloratadine, rupatadine and any other oral H1 antihistaminic not listed above (6 days), systemic corticosteroids (28 days), ketotifen (2 weeks), macrolides and imidazolic antifungals (7 days), anticholinergic drugs (7 days), topical antihistaminic drugs (e.g., nasal or drops) (2 days), drugs with antihistamine properties (e.g., phenothiazine) (6 days), intranasal/systemic decongestants and eye drops (3 days).
4. Patients suffering from non-allergic rhinitis (e.g., vasomotor, infectious, or drug-induced rhinitis).
5. Patients who had undergone nasal surgery in the previous 6 months and patients with nasal polyps, significant deviation of the nasal septum, acute or chronic sinusitis.
6. Clinically relevant respiratory tract malformations.
7. Recent nasal biopsy (within 2 months).
8. Nasal trauma; nasal surgery; atrophic rhinitis; rhinitis medicamentosa (within 2 months).
9. Antibiotic use for acute conditions within 2 weeks prior to screening.
10. History of QT prolongation and/or torsade de pointes, including congenital long QT syndromes, history of cardiac arrhythmias.
11. Smokers who reported consuming more than 10 cigarettes per day (4).
12. History or presence of an immunodeficiency disorder.
13. Hypersensitivity to any beta-agonist, sympathomimetic drug, LT antagonist.
14. Patients with a known history of human immunodeficiency virus (HIV), hepatitis B or hepatitis C infection.
15. Any abnormal laboratory value of clinical relevance.
16. Pregnant or nursing mother.
17. Current evidence of any clinically significant disease of the hematopoietic, gastrointestinal, cardiovascular, pulmonary, or any other systems that might have hindered the subject participation.
18. Abuse or dependency of alcohol, narcotics, opioids or any other addictive substances.
19. Patients who participated in any type of clinical study within the last month of the screening date.
20. Unsuitability for enrolment otherwise as decided by the investigator.
21. The drug products contain lactose monohydrate. Patients with rare hereditary problems of galactose intolerance, lactase deficiency or glucose-galactose malabsorption should not have taken these drugs.
22. Patients who had received immunotherapy for less than 6 months (unless on a stable dose within the prior month, and none within 24 hours before any study visit) or any central nervous system (CNS) acting agents (including antidepressants, sedatives, anxiolytics, hypnotics, opioids or neuroleptics) at any time.

### 4.3.1. Treatments Administered

Eligible subjects were randomly assigned in a 1:1 ratio to receive either:
- 10 mg tablet of rupatadine and 10 mg tablet of montelukast (test product group) or
- 10 mg tablet of rupatadine and 1 placebo tablet (reference product group)

The IMPs were taken orally once daily for 28 ± 2 days starting on the randomisation day. Since the IMPs were taken in the evening, they were not taken on the day of the week 4/EoT visit. The IMPs were taken orally with 240 mL of water before the meal in the evening for 28 ± 2 days starting on the randomisation day. IMP doses were taken 24 hours apart with a time window of ± 2 hours.

Rupatadine (Rupafin^{®}) 10 mg tablets and montelukast (Singulair^{®}) 10 mg film-coated tablets were administered to the test product treatment group. Rupatadine (Rupafin^{®}) 10 mg tablets and film-coated placebo tablets were administered to the reference product treatment group. To maintain the blinding throughout the study, montelukast and placebo tablets were film-coated, and placebo tablets resembled in colour and weight the montelukast tablets. Furthermore, the manufacturing batch did not appear in the label text to guarantee the blind nature of the study.

### 4.3.2. Prior and Concomitant Therapy

### 4.3.2.1. Prohibited Concomitant Therapy

1. Concomitant CYP3A4 substrates and inducers should have been avoided since rupatadine is a substrate of CYP3A4. The combination of rupatadine with potent CYP3A4 inhibitors (like oral antifungals) should have been avoided. Moderate CYP3A4 inhibitors (like erythromycin) should have been administered with caution. Dose adjustment of sensitive CYP3A4 substrates (e.g., simvastatin, lovastatin) and CYP3A4 substrates with a narrow therapeutic index (e.g., ciclosporin, tacrolimus, sirolimus, everolimus, cisapride) could have been required as rupatadine may increase plasma concentrations of these drugs.
2. Since montelukast is metabolised by CYP3A4, CYP2C8, and CYP2C9, caution should have been exercised when montelukast was coadministered with inducers of CYP3A4, CYP2C8, and CYP2C9, such as phenytoin, phenobarbital, and rifampicin. In a clinical drug-drug interaction study involving montelukast and gemfibrozil (an inhibitor of both CYP2C8 and CYP2C9), gemfibrozil increased the systemic exposure of montelukast by 4.5-fold (25). Theophylline, beta-agonists (oral or long-acting), or anticholinergics within 1 week, cromolyn sodium or nedocromil within 2 weeks, corticosteroids within last month, cimetidine hydrochloride, warfarin, digoxin, clarithromycin, erythromycin, troleandomycin, chlorpheniramine maleate, clemastine fumarate, diphenhydramine, or hydroxyzine within 2 weeks, ketoconazole or itraconazole within 7 days, loratadine or desloratadine within 6 days, cetirizine within 3 days, levocetirizine within 2 days, bilastine within 3 days, or, azithromycin within 1 month.
3. The coadministration of rupatadine or montelukast with grapefruit juice was not recommended.
4. Initiation of new immunotherapy or changes in doses during the study visits.
5. Patients taking systemic therapy for bronchial asthma or any other new asthma medications other than short-acting inhaled beta-agonists were discontinued from the study when the new therapy was instituted.
6. Anticholinergic drugs, other anti-H1 or chromones were not permitted during the study.

### 4.3.2.2. Permitted Concomitant Therapy

Subjects receiving immunotherapy for at least 6 months had to maintain therapy at a stable dose within the prior month and throughout the study visits. Inhaled or systemic corticosteroid therapy for asthma was permitted if initiated at least 4 weeks before the baseline visit and maintained at a constant dosage throughout the study.

### 4.3.2.3. Rescue Therapy

The following medications could have been provided to subjects as needed to treat their allergic symptoms of rhinoconjunctivitis during the pollen season: decongestant nasal spray or eye drops (like naphazoline) as rescue medication for a maximum of 3 days a week.

Asthmatic subjects could have taken inhaled short-acting β-2-agonist to treat their bronchial symptoms (exacerbations) caused by allergy.

### 5. TRIAL ASSESSMENTS AND PROCEDURES

The timing of the trial assessments and procedures is presented in Table 3:

**Table 3: BMI, body mass index; EoT, end of treatment; IMP, investigational medicinal product; PDC, patient diary card. a Including the prick test; b Eligibility confirmation; c If required, wash out of ongoing forbidden medications as per inclusion criteria. The wash-out period could vary from 0 to 28 days, depending on a subject's medications (see section 4.2.1); d An electrocardiogram performed within the last 3 months prior to the inclusion in the study, including all requested values per protocol, was valid at screening; e Only for women with childbearing potential; f A medication started and stopped prior to the first IMP dose was classified as a prior medication. A concomitant medication was defined as a medication that started prior to, on or after the first IMP dose and continued to be taken during the study; g Any untoward medical occurrence prior to the first IMP dose was reported as medical history; h Included in the PDCs; i T3NNSS, total three non-nasal symptom score; T4NSS, total four nasal symptom score; T7SS, total seven symptom score; j For screening nasal severity score (see section 4.2.1 for inclusion criteria), T4NSS was calculated as the sum of six (6) measurement evaluations performed 12 hours apart (evening and following morning) with at least two (2) non-consecutive measurements after completing the wash-out period. Eligible subjects had to have at least 48 out of 72 points. The six (6) measurements had to be performed within a period of four (4) to seven (7) days, preferably five (5) days before Day 1. If a patient did not reach the severity score to be eligible five (5) days before Day 1, due to an incomplete or unclear record or any other reason (e.g., visit on the weekend), two (2) additional days (i.e. up those mentioned seven (7) days period) could be assessed. In that case, the first screening visit was between Day -35 and Day -8, and the eligibility assessment was between Day -7 and Day -1; k Only for subjects with asthma.**

| **Assessment/procedure** | **Screening period** | | **Treatment period** | | | | |
|---|---|---|---|---|---|---|---|
| | **Screening Visit 1 (Day -33 to -6)** | **Screening Visit 2 (Day -5 to -1)** | **Baseline Visit (Day 1)** | **Week 1 (Day 7 ± 2)** | **Week 2 (Day 14 ± 2)** | **Week 3 (Day 21 ± 2)** | **Week 4/EoT (Day 28 ± 2)** |
| Informed consent | X | | | | | | |
| Inclusion/exclusion criteria | X^{a} | | X^{b} | | | | |
| Wash-out period^{c} | X | | | | | | |
| Demographics | X | | | | | | |
| Weight, height, and BMI | X | | | | | | |
| Relevant medical history and prior/current medications | X | | | | | | |
| Randomisation | | | X | | | | |
| Physical examination | X | | X | | | | |
| Vital sign | X | | X | X | X | X | X |
| Electrocardiogram | X^{d} | | | | | | X |
| Serum biochemistry | X | | | | | | X |
| Pregnancy test^{e} | X | | | | | | X |
| Concomitant medications^{f} | | X | X | X | X | X | X |
| Adverse events recording^{g} | | | | X | X | X | X |
| IMP dispensing | | | X | | | | |
| IMP compliance | | | | X | X | X | X |
| IMP return and accountability | | | | | | | X |
| PDC distribution | X | | X | X | X | X | X |
| PDC collection | | | X | X | X | X | X |
| Non-nasal and nasal symptoms and symptom scores^{h,i} | | X^{j} | | X | X | X | X |
| Number of asthma attacks^{h,k} | | X | | X | X | X | X |
| Number of nocturnal awakenings^{h,k} | | X | | X | X | X | X |

### 5.1. Efficacy Assessments

### 5.1.1. Patient Diary Card (PDCs)

Subjects were provided with PDCs (PDC1-PDC5) to assess the severity of:
- Nasal and non-nasal symptoms (section 5.1.2) and
- Asthma symptoms (*only for subjects with asthma;* section 5.1.3)
and to record:
- IMP intake
- Any additional medications
- AEs (see below section 5.2.1)
- Number of asthma attacks (*only for subjects with asthma),* and
- Number of nocturnal awakenings (*only for subjects with asthma*)

Subjects started completing a new PDC on each visit day (starting from the screening visit 1) and returned the completed PDC at the next study visit.

### 5.1.2. Nasal and Non-Nasal Symptom Scores

Subjects assessed nasal and non-nasal symptoms using a predefined scale twice daily (reflective evaluation over the past 12 hours): in the morning on awakening (for rating night-time nasal/non-nasal symptom scores) and in the evening before sleeping (for rating daytime nasal/non-nasal symptom scores). The evening assessment was recommended to be done before taking the IMPs.

T4NSS, T3NNSS, and T7SS were computed using a subject's nasal and non-nasal symptom ratings.

T4NSS comprised nasal symptoms: nasal blockade/congestion, rhinorrhoea (running nose), nasal itching, and sneezing. Each symptom was rated by subjects as follows:
- 0 = no symptoms
- 1 = mild symptoms (sign/symptom clearly present, but minimal awareness; easily tolerated)
- 2 = moderate symptoms (definite awareness of sign/symptom that is bothersome but tolerable)
- 3 = severe symptoms (sign/symptom that is hard to tolerate; causes interference with activities of daily living and/or sleeping)

The maximum score for T4NSS was twelve (12).

T3NNSS comprised non-nasal symptoms: pharyngeal itching, tearing, and conjunctival redness/ocular pruritus. Each symptom was rated by subjects as follows:
- 0 = no symptoms
- 1 = mild symptoms (sign/symptom clearly present, but minimal awareness; easily tolerated)
- 2 = moderate symptoms (definite awareness of sign/symptom that is bothersome but tolerable)
- 3 = severe symptoms (sign/symptom that is hard to tolerate; causes interference with activities of daily living and/or sleeping)

The maximum score for T3NNSS was nine (9).

T7SS included both nasal (T4NSS) and non-nasal symptoms (T3NNSS). The maximum score for T7SS was 21.

DSS (Daily Score Symptoms) was calculated for each symptom as the average of the measurements performed in the evening and the following morning. DSS ranged thus from 0 to 3. DSS was only calculated if both the evening and morning evaluations were available. If a subject provided 2 repeated assessments for the same evening or morning, the first assessment was used.

### 5.1.3. Assessments Related to Asthma

Subjects with asthma completed a daytime symptom diary scale (included in the PDC) to assess the frequency of general asthma symptoms, inconvenience of asthma symptoms, frequency of limitation during usual activities, and frequency with which asthma symptoms limited the ability to perform usual activities.

In addition, subjects with asthma recorded the daily number of asthma attacks and nocturnal awakenings in the PDC.

### 5.2. Safety Assessment

### 5.2.1. Adverse Events (AE)

The investigator and the site staff were responsible for detecting and reporting all AEs regardless of whether they were spontaneously reported, elicited, or based on signs and symptoms recorded in the PDC. All AEs, regardless of a causal relationship with the trial treatment and including any AEs of special interest, had to be registered and documented in *the Adverse Events* eCRF by the investigator. If an abnormal laboratory test result was related to clinical signs or symptoms requiring therapeutic intervention or further diagnostics, the diagnosis or medical condition was entered in the subject's *Adverse Events* eCRF. If the laboratory abnormality was the primary reason for an unforeseen hospitalisation or otherwise fulfilled the seriousness category of an AE, the procedure for rapid notification of SAEs had to be followed. Likewise, if the laboratory abnormality led to discontinuation of the subject from the study, the subject was followed until the abnormality was resolved or stabilised.

The following was recorded on each AE: description, start and end dates, seriousness, causality, severity/intensity, action taken with the IMP, other actions, and outcome.

The investigator and the Sponsor assessed the *seriousness and causality* (with the IMP) of AEs. Causality was categorised as definitive, probable, possible, conditional, not related, or non-assessable. Definitive, probable, possible, conditional, and non-assessable AEs were considered IMP-related. The investigator assessed the *severity*/*intensity* of AEs as mild, moderate, or severe.

### 5.2.2. Vital Signs and ECG

Heart rate, respiratory rate, and systolic and diastolic blood pressure were measured with the subject in a sitting position. The investigator assessed the results as normal, abnormal not clinically significant, or abnormal clinically significant. Standard 12-lead ECG (with QT interval, QTc interval, QTcF interval, T wave, and heart rate) was recorded, and the investigator evaluated the assessment as normal, abnormal not clinically significant, or abnormal clinically significant. An ECG performed within 3 months prior to including a subject in the study was accepted for screening if QT, QTc, and QTcF interval values were available.

### 5.2.3. Laboratory Assessments

Blood samples for serum biochemistry were collected and the following parameters were assessed: total protein, albumin, alkaline phosphatase, aspartate aminotransferase (AST), alanine aminotransferase (ALT), gamma-glutamyl transferase (GGT), total bilirubin, triglycerides, total cholesterol, glucose, calcium, potassium, sodium, urea, creatinine, glomerular filtration rate (GFR), and uric acid. If a serum biochemistry value was preceded by < or > sign (i.e., a value below or above quantification limits), the investigator was instructed to enter in the eCRF the quantification limit. The investigator evaluated each assessment as normal, abnormal not clinically significant, or abnormal clinically significant. Urine or serum pregnancy test was performed for women with childbearing potential.

Laboratory analyses were performed at each study site's local laboratory.

### 5.3. Primary Efficacy Variable

The primary efficacy endpoint was the change from baseline in the mean T4NSS over the treatment period (reflective 12 hours symptoms, every day including the visit days with the investigator). Baseline score was calculated as the mean of the score provided by the investigator at screening (screening nasal severity score).

### 6. STATISTICAL METHOD AND ANALYSES

### 6.1.1. Efficacy Analysis - analysis of the Primary Endpoint

The primary efficacy endpoint was the change in the mean T4NSS from the baseline over the treatment period. The global null hypothesis was that the combination of rupatadine and montelukast does not differ from or is inferior to rupatadine monotherapy. The alternative hypothesis was that the combination of rupatadine and montelukast is superior to rupatadine monotherapy.

The T4NSS change from the baseline over the treatment period was calculated as follows:$T 4 NSS change = mean T 4 NSS - mean T 4 NSS at baseline \left(as per investigator\right)$

**DSS** (Daily Score Symptoms) was calculated for each symptom as the average of the measurements performed in the evening and the following morning. DSS ranged thus from 0 to 3. DSS was only calculated if both the evening and morning evaluations were available. If a subject provided 2 repeated assessments for the same evening or morning, the first assessment was used.

**T4NSS** was calculated as the sum of all nasal DSSs during the treatment period. T4NSS was available for each day and ranged from 0 to 12. The score was calculated if the DSS was available for all 4 nasal symptoms.

**Mean T4NSS** was calculated as the mean of T4NSSs over the treatment period (from the first to the last treatment day). There was one mean T4NSS per subject ranging from 0 to 12.

**Screening nasal severity score** was the sum of six (6) measurement evaluations performed 12 hours apart (evening and following morning) with at least two (2) non-consecutive measurements after completing the wash-out period. Eligible subjects had to have at least **48 out of 72 points.** The six (6) measurements had to be performed within a period of four (4) to seven (7) days, preferably five (5) days before Day 1. The investigator recorded the score in the *Symptom Scores* eCRF at the screening visit; the score was used as an inclusion criterion.

**Mean T4NSS at baseline** (as per investigator) was calculated as the screening nasal severity score divided by 6.

Differences between the treatment groups were assessed using an analysis of covariance (ANCOVA) model adjusted by treatment and site as factors and the mean T4NSS at baseline (as per investigator) as a covariate.

The following **supplementary analyses** were performed for the primary efficacy endpoint:
- Change from baseline (as per investigator) in the mean T4NSS over the first 7 days, first 14 days, and first 21 days of the treatment period.
- Change from baseline (calculated) in the mean T4NSS over the treatment period (reflective evaluation)

### 6.1.2. Efficacy Analysis - analysis of the Secondary Endpoints

The changes in T3NNSS, T7SS, and DSSs from the baseline over the treatment period were calculated similarly to T4NSS. The same morning and evening assessments used for recalculating the baseline (calculated) mean T4NSS were used for calculating the baseline mean T3NNSS, T7SS, and DSSs. Differences between the treatment groups from the baseline over the treatment period were analysed using the same ANCOVA model as for the primary endpoint.

### 6.1.3. Efficacy Analysis - analysis of the Exploratory Endpoints

The number of asthma attacks, and nocturnal awakenings were listed and tabulated. The baseline number of asthma attacks and nocturnal awakenings were calculated as the average over the screening period. The number of asthma attacks, and nocturnal awakenings over the treatment period were calculated as the average of all available days from Day 1 to the end of the treatment period. Differences between the treatment groups were analysed using the same ANCOVA model as for the primary endpoint.

### 6.1.4. Safety Analyses

### Treatment Exposure and Compliance

IMP accountability (i.e., IMP dispensing and return) per subject was listed. Treatment exposure and compliance were listed and summarised by treatment. The exposure was presented as the number of tablets taken during the treatment period; the duration of exposure was presented in days. The treatment compliance (%) was calculated as the number of tablets taken (both pink and beige) divided by the number of tablets expected to have been taken from the IMP dispensation date to the date of the last dose +1 multiplied by 100. Non-compliant subjects (defined as subjects who missed any of the two tablets a minimum of 8 non-consecutive days or 6 consecutive days) were listed and tabulated.

### AEs

AEs were coded using the MedDRA version 23.1. AE listing included the original term, PT, SOC, start and end dates, duration, severity, seriousness, causal relationship (with the IMP), actions taken, and outcome. A separate listing was presented for SAEs.

The count and percentage of subjects with at least one AE were summarised and sorted by severity/intensity, seriousness, and causality. AEs were also summarised by SOC and PT (counted by subject and event). Subjects were counted only once for each SOC and PT. If more than one AE within the same SOC/PT was reported for a subject, the AE with the worst-case severity or causality was included in the corresponding summaries. SAEs were summarised separately.

### Vital Signs and ECG

Values for vital signs (heart rate, respiratory rate, and systolic and diastolic blood pressure) and clinical assessments (as per investigator) were listed. Values for the baseline and week 4/EoT visits and the changes from the baseline to week 4/EoT were summarised. In addition, visit summaries of clinical assessments were provided.

ECG measurements and clinical assessments (as per investigator), as well as changes in QT, QTc and QTcF intervals, T wave, and heart rate, were listed by subject and summarised by visit. In addition, QT and QTcF intervals were categorised (< 30 ms increase, 30-60 ms increase, > 60 ms increase, and decrease from the baseline) and summarised to evaluate markedly abnormal changes from the screening 1 visit to the week 4/EoT visit. To evaluate clinically significant values, QT and QTcF intervals were categorised (≤ 450 ms and > 450 ms) and summarised for the screening 1 and week 4/EoT visits.

### 7. RESULTS

### 7.1. Trial Subjects

A total of 335 adults with moderate to severe SAR and with or without mild or moderate bronchial asthma were screened. Of these patients, 301 (89.9%) were randomly assigned to one of the two treatment groups: 149 (49.5%) to the reference product group (rupatadine 10 mg plus placebo) and 152 (50.5%) to the test product group (rupatadine 10 mg plus montelukast 10 mg).

There were 34 screen failures (10.1% of all screened patients). The most common reason for screen failure was not meeting the eligibility criteria (76.5% of screen failures). Four (4) subjects (2.7%) in the reference product group and four (4) subjects (2.6%) in the test product group did not initiate the trial treatment. Eleven (11) subjects (7.4%) in the reference product group and seven (7) subjects (4.6%) in the test product group discontinued the trial prematurely. There were no reports of the subjects being unblinded during the trial. Table 4 summarises the disposition of all subjects and the reasons for screen failures and premature termination.

Of the 335 screened patients, 169 (50.4%) were screened in Poland (6 sites), 93 (27.8%) in Bulgaria (4 sites), and 73 (21.8%) in Spain (11 sites). There were 19 screen failures in Poland, 14 in Spain, and 1 in Bulgaria. Of the 301 randomised subjects, 150 (49.8%) were from Poland, 92 (30.6%) from Bulgaria, and 59 (19.6%) from Spain.

**Table 4: Subject Disposition by Assigned Treatment (All Subjects)**

| **Disposition** | **Rupatadine 10 mg + Placebo n (%)** | **Rupatadine 10 mg + Montelukast 10 mg n (%)** | **All n (%)** |
|---|---|---|---|
| **Screened** | - | - | 335 |
| **Randomised** | 149 (100) | 152 (100) | 301 (100) |
| **Treated** | 145 (97.3) | 148 (97.4) | 293 (97.3) |
| **Completed visits** | - | - | - |
| Screening 1 | 149 (100) | 152 (100) | 301 (100) |
| Screening 2 | 149 (100) | 152 (100) | 301 (100) |
| Baseline | 149 (100) | 152 (100) | 301 (100) |
| Week 1 | 145 (97.3) | 148 (97.4) | 293 (97.3) |
| Week 2 | 139 (93.3) | 147 (96.7) | 286 (95.0) |
| Week 3 | 138 (92.6) | 145 (95.4) | 283 (94.0) |
| Week 4 | 138 (92.6) | 145 (95.4) | 283 (94.0) |
| **Completed trial** | 138 (92.6) | 145 (95.4) | 283 (94.0) |
| **Premature termination** | 11 (7.4) | 7 (4.6) | 18 (6.0) |
| **Screen failures** | - | - | 34 |
| Inclusion/exclusion criteria | - | - | 26 |
| Subject decision | - | - | 3 |
| Competing trial | - | - | 0 |
| Other | - | - | 5 |
| **Premature termination** | 11 (100) | 7 (100) | 18(100) |
| Adverse event | 1 (9.1) | 0 | 1 (5.6) |
| Protocol violation | 0 | 0 | 0 |
| Withdrawal by subject | 3 (27.3) | 0 | 3 (16.7) |
| Lost to follow-up | 0 | 0 | 0 |
| Concomitant disease | 0 | 0 | 0 |
| Lack of efficacy | 1 (9.1) | 1 (14.3) | 2(11.1) |
| Other | 6 (54.5) | 6 (85.7) | 12 (66.7) |

Table 5 presents the number and percentage of subjects included in and excluded from each analysis set.

**Table 5: Analysis Populations (All Randomised Subjects)**

| **Analysis Population** | **Rupatadine 10 mg + Placebo N=149 n (%)** | **Rupatadine 10 mg + Montelukast 10 mg N=152 n (%)** | **All N=301 n (%)** |
|---|---|---|---|
| **Safety** | | | |
| Subjects included | 145 (97.3) | 148 (97.4) | 293 (97.3) |
| Subjects excluded | 4 (2.7) | 4 (2.6) | 8 (2.7) |

| **Intention-to-treat** | | | |
|---|---|---|---|
| Subjects included | 143 (96.0) | 147 (96.7) | 290 (96.3) |
| Subjects excluded | 6 (4.0) | 5 (3.3) | 11 (3.7) |

| **Per Protocol** | | | |
|---|---|---|---|
| Subjects included | 127 (85.2) | 128 (84.2) | 255 (84.7) |
| Subjects excluded | 22 (14.8) | 24 (15.8) | 46 (15.3) |

In total, 293 SAR patients (133 men [45.4%] and 160 women [54.6%]) aged 18 to 77 years (mean age 38.7 years) participated in the treatment phase of this trial (i.e., received at least one IMP dose). There were more women (58.6%) than men (41.4%) in the reference product group, whereas the sex ratio was equal (50.7% women vs 49.3% men) in the test product group. There were slightly more overweight and obese subjects in the test product group (40.0% and 18.5%, respectively) than in the reference product group (35.8% and 14.9%, respectively). Otherwise, the treatment groups were similar in demographics and anthropometrics. Furthermore, the safety, ITT, and PP populations were similar in demographics and anthropometrics.

No differences in vital signs (blood pressure, heart rate, and respiratory rate) were observed between the treatment groups at the baseline visit. There were more abnormal clinically significant eye examination assessments in the reference product group (26.2%) than in the test product group (18.9%). Otherwise, the physical examination results were similar between the groups at the baseline visit.

A total of 18.1% of the safety population had asthma. The percentage was slightly higher in the reference product group (20.0%; 29/145 subjects) than in the test product group (16.2%; 24/148 subjects).

Overall treatment compliance was 98.7% (the safety population). There was no difference between the treatment groups: the compliance was 98.8% in the reference product group and 98.7% in the test product group. A total of 95.9% (281/293 subjects) of the subjects in the safety population were compliant; 94.5% (137/145 subjects) of the reference product group and 97.3% (144/148 subjects) of the test product group were compliant.

### 7.2. Efficacy results

### 7.2.1. Primary endpoint

The changes in the mean T4NSS from the baseline (as per investigator) over the treatment period are presented for the ITT population in Table 6. For the ITT population, the least squares (LS) mean change was -5.37 (95% confidence interval [CI] -5.84 to -4.91) in the reference product group and -5.81 (95% CI -6.27 to -5.36) in the test product group. This demonstrates a difference of -0.44 (95% CI -0.90 to 0.02) in the LS mean difference (or -4.5% mean difference) for the test product compared to the reference product. The difference showed a trend that the test product (rupatadine 10 mg plus montelukast 10 mg) is **superior** in reducing all T4NSS symptoms compared to the reference product (rupatadine 10 mg plus placebo).

**Table 6: Change in the Mean T4NSS from the Baseline (as per Investigator) over the Treatment Period (ITT Population)**

| **Variable** | **Statistics** | **Rupatadine 10 mg + Placebo (N=143)** | **Rupatadine 10 mg + Montelukast 10 mg (N=147)** |
|---|---|---|---|
| **Mean T4NSS at the baseline** | n | 143 | 147 |
| | Mean | 9.64 | 9.63 |
| | SD | 1.09 | 1.15 |
| | Median | 9.50 | 9.50 |
| | 25^{th} %ile, 75^{th} %ile | 8.67, 10.50 | 8.67, 10.50 |
| | Min, max | 7.7, 12.0 | 7.3, 12.0 |
| **Mean T4NSS over the treatment period** | n | 143 | 147 |
| | Mean | 4.07 | 3.64 |
| | SD | 2.36 | 2.26 |
| | Median | 3.73 | 3.20 |
| | 25th %ile, 75th %ile | 2.26, 5.66 | 1.94, 5.14 |
| | Min, max | 0.1, 11.9 | 0.0, 10.9 |
| **Change from the baseline over the treatment period** | n | 143 | 147 |
| | Mean | -5.57 | -6.00 |
| | SD | 2.47 | 2.31 |
| | Median | -5.70 | -6.37 |
| | 25th %ile, 75th %ile | -7.39, -4.21 | -7.70, -4.52 |
| | Min, max | -10.50, 2.50 | -10.40, 0.40 |
| | LS Mean | **-5.37** | **-5.81** |
| | 95% CI | -5.84 to -4.91 | -6.27 to -5.36 |
| **Group difference** | Mean difference | - | **-4.5%** |
| | LS mean difference | - | **-0.44** |
| | 95% CI | - | -0.90 to 0.02 |
| | *P value* | - | .060 |

| | | | |
|---|---|---|---|
| CI, confidence interval; ITT, intention-to-treat; LS, least squares; Max, maximum, Min, minimum; SD, standard deviation; T4NSS, total four nasal symptom score. | | | |

As a supplementary analysis, change in the mean T4NSS was analysed from the baseline (as per investigator) over the first 7, 14, and 21 days of treatment. For the ITT population, the mean T4NSS at baseline was 9.64 (SD 1.09) for the reference product group and 9.63 (SD 1.15) for the test product group. The reduction in the mean T4NSS was **greater** for the test product than for the reference product over the first 7, 14, and 21 days, but the difference between the groups remained the same after 14 days (Table 7).

**Table 7: Change in the Mean T4NSS from the Baseline (as per Investigator) over the First 7, 14, and 21 Days of Treatment (ITT Population)**

| **Variable** | **Treatment** | **Statistics** | **First 7 Days of Treatment** | **First 14 Days of Treatment** | **First 21 Days of Treatment** |
|---|---|---|---|---|---|
| **Change from the baseline** | Rupatadine 10 mg + Placebo (N=143)^{a} | LS mean (95% CI) | -4.01 (-4.58 to -3.45) | -4.50 (-5.02 to -3.98) | -4.99 (-5.48 to -4.49) |
| | | SD | 2.775 | 2.670 | 2.604 |
| | Rupatadine 10 mg + Montelukast 10 mg (N=147)^{a} | LS mean (95% CI) | -4.44 (-4.99 to -3.89) | -4.95 (-5.45 to -4.44) | -5.44 (-5.92 to -4.96) |
| | | SD | 2.606 | 2.525 | 2.445 |
| **Difference between the treatment groups** | - | Mean difference | **-4.4%** | **-4.7%** | **-4.7%** |
| | - | LS mean difference | -0.42 | -0.45 | -0.45 |
| | - | 95% CI | -0.98 to 0.14 | -0.96 to 0.06 | -0.94 to 0.03 |
| | - | *P value* | .138 | .086 | .067 |

| | | | | | |
|---|---|---|---|---|---|
| CI, confidence interval; ITT, intention-to-treat; LS, least squares; SD, standard deviation; T4NSS, total four nasal symptom score. ^{a}Same as n for each analysis. | | | | | |

As set out in Table 7, encouragingly, the test product group demonstrated a **-4.4%** mean difference for all T4NSS symptoms compared to the reference product after the first 7 days of treatment; a **-4.7%** mean difference for all T4NSS symptoms compared to the reference product after the first 14 days of treatment; and a **-4.7%** mean difference for all T4NSS symptoms compared to the reference product after the first 21 days of treatment.

### 7.2.2. Secondary Endpoints

### 7.2.2.1. Changes in the Mean T3NNSS and T7SS from the Baseline (Calculated) over the Treatment Period

Changes in the mean T3NNSS and T7SS from the baseline (calculated) over the treatment period are summarised for the ITT population in Table 8. A trend that the test product is **superior** to the reference product in reducing symptoms was observed for both endpoints.

**Table 8: Changes in the Mean T3NNSS and T7SS from the Baseline (Calculated) over the Treatment Period (ITT Population)**

| **Variable** | **Treatment** | **Statistics** | **T3NNSS** | **T7SS** |
|---|---|---|---|---|
| **Change from the baseline (calculated)** | Rupatadine 10 mg + Placebo (N=143^{a}) | LS mean (95% CI) | -2.20 (-2.50 to -1.90) | -7.99 (-8.70 to -7.28) |
| | Rupatadine 10 mg + Montelukast 10 mg (N=147^{a}) | LS mean (95% CI) | -2.40 (-2.69 to -2.12) | -8.58 (-9.27 to -7.89) |
| **Difference between the treatment groups** | - | Mean difference | -3.3% | -4.0% |
| | - | LS mean difference | -0.21 | -0.59 |
| | - | 95% CI | -0.50 to 0.09 | -1.29 to 0.12 |
| | - | *P value* | .168 | .102 |

| | | | | |
|---|---|---|---|---|
| CI, confidence interval; ITT, intention to treat; LS, least squares; T3NNSS, total three non-nasal symptom score; T7SS, total seven symptom score. ^{a}Same as n for both analyses. | | | | |

### 7.2.2.2. Change in the Mean DSS for Each Symptom from the Baseline over the Treatment Period

Table 9 summarises the changes in the mean DSSs for individual nasal and non-nasal symptoms from the baseline over the treatment period. The test product was **more effective** than the reference product in reducing **sneezing** with a statistically significant difference: a difference of -0.16 (95% CI -0.28 to -0.05; P=.007) was found in the LS mean difference (or -6.7% mean difference) for the test product compared to the reference product. A similar trend was observed for the other symptoms.

**Table 9: Changes in the Mean Daily Symptom Scores for Individual Nasal and Non-Nasal Symptoms from the Baseline (Calculated) over the Treatment Period (ITT Population)**

| **Variable** | **Treatment** | **Statistics** | **Nasal Blockadel Congestion** | **Rhinorrhoea** | **Nasal Itching** | **Sneezing** | **Pharyngeal Itching** | **Tearing** | **Conjunctival Redness/ Ocular Pruritus** |
|---|---|---|---|---|---|---|---|---|---|
| **Change from the baseline (calculated)** | Rupatadine 10 mg + Placebo (N=143^{a}) | LS mean (95% CI) | -1.33 (-1.47 to -1.19) | -1.47 (-1.60 to -1.33) | -1.51 (-1.64 to -1.39) | -1.40 (-1.52 to -1.28) | -0.65 (-0.78 to -0.53) | -0.74 (-0.85 to -0.63) | -0.78 (-0.89 to -0.67) |
| | Rupatadine 10 mg + Montelukast 10 mg (N=14₇a) | LS mean (95% CI) | -1.40 (-1.54 to -1.27) | -1.59 (-1.72 to -1.46) | -1.59 (-1.71 to -1.47) | -1.56 (-1.68 to -1.45) | -0.76 (-0.88 to -0.64) | -0.82 (-0.93 to -0.72) | -0.81 (-0.92 to -0.70) |
| **Difference between the treatment groups** | - | Mean difference | **-2.7%** | **-5.1%** | **-2.7%** | **-6.7%** | **-3.8%** | **-5.0%** | **-0.3%** |
| | - | LS mean difference | -0.08 | -0.12 | -0.07 | -0.16 | -0.11 | -0.09 | -0.03 |
| | - | 95% CI | -0.21 to 0.06 | -0.25 to 0.01 | -0.20 to 0.05 | -0.28 to - 0.05 | -0.23 to 0.01 | -0.19 to 0.02 | -0.14 to 0.08 |
| | - | *P value* | .287 | .079 | .246 | **.007** | .085 | .113 | .592 |

| | | | | | | | | | |
|---|---|---|---|---|---|---|---|---|---|
| CI, confidence interval; ITT, intention to treat; LS, least squares. ^{a}Same as n for each analysis. | | | | | | | | | |

### 7.2.2.3. Onset of Action for T4NSS, T3NNSS, T7SS, and individual symptoms (DSS)

For all individual DSSs, T4NSS, T3NNSS, and T7SS, the onset of action was on Day 1 (P<.05). Statistically significant differences between the treatment groups were found for sneezing starting on Day 4 (P=.038) (Table 10) and for rhinorrhoea starting on Day 8 (P=.041) (Table 11).

**Table 10. Onset of action for sneezing. ITT population.**

| | **Rupatadine 10 mg + Placebo (N=143)** | | | **Rupatadine 10 mg + Montelukast 10mg (N=147)** | | | |
|---|---|---|---|---|---|---|---|
| | Daily | Cumulative | | Daily | Cumulative | | |
| Day | Mean (SD) | Mean (SD) | p-value# | Mean (SD) | Mean (SD) | p-value# | p-value## |
| Baseline | 2.4 (0.57) | 2.4 (0.57) | - | 2.3 (0.52) | 2.3 (0.52) | - | - |
| Day 1 | 1.6 (0.81) | 1.6 (0.81) | <.0001 | 1.4(0.81) | 1.4(0.81) | <.0001 | 0.3534 |
| Day 2 | 1.5 (0.88) | 1.5 (0.79) | <.0001 | 1.2 (0.78) | 1.3 (0.74) | <.0001 | 0.1350 |
| Day 3 | 1.4 (0.87) | 1.5 (0.78) | <.0001 | 1.1 (0.81) | 1.3 (0.71) | <.0001 | 0.0687 |
| **Day 4** | 1.3 (0.83) | 1.4 (0.76) | <.0001 | 1.1 (0.81) | 1.2 (0.71) | <.0001 | **0.0382** |

| | | | | | | | |
|---|---|---|---|---|---|---|---|
| The cumulative mean at baseline is the same value as the daily mean. The cumulative mean of each day is the average assessment from Day-1 to that Day. P-value# resulted from the comparison of the cumulative mean at each day with the baseline mean of the same group by means of Wilcoxon Signed-Rank test. P-value## resulted from the comparison of the change from baseline of the cumulative mean at each day between treatment groups by means of Mann-Whitney U test. | | | | | | | |

**Table 11. Onset of action for rhinorrhea. ITT population.**

| | **Rupatadine 10 mg + Placebo (N=143)** | | | **Rupatadine 10 mg + Montelukast 10mg (N=147)** | | | |
|---|---|---|---|---|---|---|---|
| | Daily | Cumulative | | Daily | Cumulative | | |
| Day | Mean (SD) | Mean (SD) | p-value# | Mean (SD) | Mean (SD) | p-value# | p-value## |
| Baseline | 2.6 (0.36) | 2.6 (0.36) | - | 2.6 (0.38) | 2.6 (0.38) | - | - |
| Day 1 | 1.7 (0.82) | 1.7 (0.82) | <.0001 | 1.7 (0.86) | 1.7 (0.86) | <.0001 | 0.7520 |
| Day 2 | 1.7 (0.88) | 1.7 (0.81) | <.0001 | 1.5 (0.85) | 1.6 (0.80) | <.0001 | 0.3987 |
| Day 3 | 1.6 (0.84) | 1.7 (0.77) | <.0001 | 1.4 (0.88) | 1.5 (0.79) | <.0001 | 0.2176 |
| Day 4 | 1.6 (0.88) | 1.6 (0.77) | <.0001 | 1.3 (0.91) | 1.5 (0.80) | <.0001 | 0.0696 |
| Day 5 | 1.5 (0.89) | 1.6 (0.77) | <.0001 | 1.3 (0.88) | 1.5 (0.79) | <.0001 | 0.0596 |
| Day 6 | 1.4 (0.90) | 1.6 (0.77) | <.0001 | 1.3 (0.92) | 1.4 (0.79) | <.0001 | 0.0680 |
| Day 7 | 1.4 (0.86) | 1.5 (0.76) | <.0001 | 1.3 (0.87) | 1.4 (0.78) | <.0001 | 0.0526 |
| **Day 8** | 1.4 (0.89) | 1.5 (0.76) | <.0001 | 1.2 (0.85) | 1.4 (0.77) | <.0001 | **0.0406** |

| | | | | | | | |
|---|---|---|---|---|---|---|---|
| The cumulative mean at baseline is the same value as the daily mean. The cumulative mean of each day is the average assessment from Day-1 to that Day. P-value# resulted from the comparison of the cumulative mean at each day with the baseline mean of the same group by means of Wilcoxon Signed-Rank test. P-value## resulted from the comparison of the change from baseline of the cumulative mean at each day between treatment groups by means of Mann-Whitney U test. | | | | | | | |

### 7.2.2.4. Percentage of Subjects Requiring Rescue Medication

Three (3) subjects in the ITT population required rescue medication during the trial: 1/143 subjects (0.7%) in the reference product group and 2/147 subjects (1.4%) in the test product group (P=1.000, Fisher's Exact Test).

### 7.2.3. Exploratory Endpoints

### 7.2.3.1. Change in the Mean Total Number of Asthma Attacks from the Baseline over the Treatment Period

Change in the mean total number of asthma attacks from the baseline over the treatment period was analysed on subjects with asthma (ITT population). The LS mean change was -0.16 (95% CI -0.30 to -0.03) in the reference product group (n=25) and -0.26 (95% CI -0.39 to -0.13) in the test product group (n=21), demonstrating a difference of -0.09 (95% CI -0.25 to 0.07; *P*=.243) in the LS mean difference for the test product compared to the reference product.

### 7.2.3.2. Change in the Mean of Nocturnal Awakenings from the Baseline over the Treatment Period

Change in the mean of nocturnal awakenings from the baseline over the treatment period was analysed on subjects with asthma (ITT population). The LS mean change was -0.35 (95% CI -0.58 to -0.12) in the reference product group (n=25) and -0.57 (95% CI -0.80 to -0.35) in the test product group (n=21). This demonstrates a difference of -0.22 (95% CI -0.49 to 0.05; P=.113) in the LS mean difference for the test product compared to the reference product.

### 7.2.3.3. Medication Use for Asthma

The use of anti-asthma medication was assessed on subjects with asthma (ITT population): 44.8% (13/29 subjects) of the reference product group and 33.3% (8/24 subjects) of the test product group used anti-asthma medication.

### 7.2.4. Efficacy Conclusions

The primary efficacy variable was the change in the mean T4NSS from the baseline (as per investigator) over the treatment period. It was observed that there is a trend for the combination of rupatadine and montelukast to be superior to rupatadine monotherapy in reducing all T4NSS symptoms (LS mean difference -0.44 or mean difference -4.5%). There was a clear trend towards superiority of the combination of rupatadine and montelukast over rupatadine monotherapy in the ITT population (LS mean difference -0.44; 95% CI -0.90 to 0.02; *P*=.060). Furthermore, the statistical significance for this superiority improved with time, as shown by the supplementary analyses (on the ITT population) assessing the change in the mean T4NSS from the baseline (as per investigator) over the first 7, 14, and 21 treatment days (for the first 7 days 95% CI -0.98 to 0.14; *P*=.138; for the first 14 days 95% CI -0.96 to 0.06; *P*=.086, and for the first 21 days 95% CI -0.94 to 0.03; *P*=.067).

The combination of rupatadine and montelukast was superior to rupatadine monotherapy for the changes in the mean T3NNSS and T7SS from the baseline (calculated) over the treatment period. Furthermore, when assessing the nasal and non-nasal symptoms separately (on the ITT population), the combination of rupatadine and montelukast was more effective than rupatadine monotherapy in reducing sneezing showing a statistically significant difference (LS mean difference -0.16; 95% CI -0.28 to -0.05; *P*=.007). A similar trend was observed for the other symptoms.

Moreover, for all individual DSSs, T4NSS, T3NNSS, and T7SS, the onset of action was on Day 1 (*P*<.05). Statistically significant differences between the treatment groups were found for sneezing starting on Day 4 (*P*=.038) and for rhinorrhoea starting on Day 8 (*P*=.041), both symptoms included in the T4NSS. In particular, these data indicate that treatment is particularly effective in the first 1 to 7, and 7 to 14 days of treatment.

Only three subjects in the ITT population required rescue medication during the trial: one subject administered with rupatadine monotherapy and two subjects with the combination of rupatadine and montelukast.

In asthma patients, the combination of rupatadine and montelukast was superior to rupatadine monotherapy for the change in the mean total number of asthma attacks and in the mean of nocturnal awakenings.

### 7.3. Safety Evaluation

### 7.3.1. Adverse Events (AEs)

A total of sixty-one (61) AEs were reported for thirty-two (32) subjects (10.9% of the safety population). A summary of subjects with AEs is presented in Table 12. There were more subjects with mild AEs (21/293 subjects; 7.2%) than with moderate AEs (13/293 subjects; 4.4%). Only two (2) subjects (0.7%) were reported to have severe AEs. Thirteen (13) subjects (4.4%) had an IMP-related AE. One (1) AE in the reference product group led to permanent treatment discontinuation. No SAEs were reported, and no deaths occurred during the trial. There were four (4) ongoing, not recovered or not resolved AEs at the end of the trial; the rest of the AEs were recovered or resolved.

**Table 12: Summary of Subjects with Adverse Events (Safety Population)**

| **Category** | **Rupatadine 10 mg + Placebo (N=145) n (%)** | **Rupatadine 10 mg + Montelukast 10 mg (N=148) n (%)** | **All (N=293) n (%)** |
|---|---|---|---|
| Subjects with AEs | 16(11.0) | 16(10.8) | 32 (10.9) |
| Subjects with mild AEs | 9 (6.2) | 12 (8.1) | 21 (7.2) |
| Subjects with moderate AEs | 7 (4.8) | 6 (4.1) | 13 (4.4) |
| Subjects with severe AEs | 2 (1.4) | 0 | 2(0.7) |
| Subjects with IMP-related AEs^{a} | 8 (5.5) | 5 (3.4) | 13 (4.4) |
| Subjects with SAEs | 0 | 0 | 0 |
| Subjects with AEs leading to death | 0 | 0 | 0 |

| | | | |
|---|---|---|---|
| AE, adverse event; IMP, investigational medicinal product; SAE, serious adverse event. ^{a}AEs with any of the following causality assessments: definitive, probable, possible, conditional, and non-assessable. | | | |

### 7.3.2. Safety Conclusions

A total of sixty-one (61) AEs were reported for 10.9% of the safety population. The percentage of subjects with at least one AE was similar between the treatment groups: 11.0% for rupatadine monotherapy and 10.8% for the combination of rupatadine and montelukast. Twenty-four (24) AEs (39.3% of all AEs) were reported for the rupatadine monotherapy group and thirty-seven (37) AEs (60.7% of all AEs) for the combination of rupatadine and montelukast group. The most common SOCs for AEs were *nervous system disorders* and *infections and infestations.* The most common PTs were *somnolence (2.1 %* of the subjects administered with rupatadine monotherapy and 2.7% with rupatadine and montelukast) and *headache* (0.7% and 2.7%, respectively).

Almost all AEs were mild or moderate in severity. Only three (3) severe AEs (not IMP-related) were reported for 2 subjects administered with rupatadine monotherapy. Eight (8) subjects (5.5%) administered with rupatadine monotherapy and five (5) subjects (3.4%) with the combination of rupatadine and montelukast had at least one IMP-related AE. No deaths occurred during the trial.

No relevant differences between the treatment groups or time-related trends within the treatment groups were observed for the serum biochemistry analytes, vital signs, and ECG variables, and there were no clinically significant abnormal assessments. Regarding the ECG parameters, there were no relevant differences between the treatment groups in the percentage of subjects who experienced an increase in QT or QTcF intervals. The percentage of subjects who experienced an increase of > 60 ms in QT interval or QTcF interval was ≤ 2% in both treatment groups. Furthermore, the percentage of subjects with values > 450 ms for QT or QTcF intervals was low (< 1% and < 3%, respectively) in both treatment groups at week 4/EoT.

Furthermore, although it has been documented that prolonged administration of montelukast may lead to adverse events including psychiatric problems, no such issues were observed in the present study in patients receiving the combination of rupatadine and montelukast.

In conclusion, the safety assessments did not raise any safety concerns. The safety profile of the combination of rupatadine and montelukast was comparable to that of rupatadine monotherapy.

### Example 3 - Post-hoc analysis

Results from patients of phase 2 study were analysed according to different sub-groups.

### 3.1. Change from the baseline of the mean T4NSS over the treatment period for patients having T4NSS ≥ 48 to ≤ 57 at screening (ITT population, ANCOVA analysis)

Changes from the baseline of the mean T4NSS over the treatment period for patients having T4NSS ≥ 48 to < 57 at screening (i.e. fulfilling the inclusion criteria of a total score of ≥ 48 (out of 72)) were analysed/extracted from the phase 2 results.

In this patient's sub-group (T4NSS ≥ 48 to < 57 at screening), the least squares (LS) mean change was -4.73 (95% CI -5.28 to -4.18) in the reference product group, and -5.32 (95% CI -5.86 to -4.78) in the test product group. This demonstrates a difference of -0.59 (95% CI -1.36 to 0.18) in the LS mean difference for the test product compared to the reference product. See below Tables 13 and 14.

**Table 13: Type III Model ANOVA for Change from the baseline of the mean T4NSS over the treatment period for patients having T4NSS ≥ 48 to < 57 at screening (ITT population, ANCOVA analysis)**

| **Source** | **Degrees of freedom** | **Sum of squares** | **Mean Square** | **F Value** | **p-value** |
|---|---|---|---|---|---|
| Mean T4NSS at Baseline | 1 | 14.862 | 14.862 | 2.83 | 0.0947 |
| Treatment | 1 | 12.034 | 12.034 | 2.29 | 0.1323 |

**Table 14: Change from the baseline of the mean T4NSS over the treatment period for patients having T4NSS ≥ 48 to < 57 at screening (ITT population, ANCOVA analysis)**

| **Variable** | **Statistics** | **Rupatadine 10 mg + Placebo (N=68)** | **Rupatadine 10 mg + Montelukast 10 mg (N=70)** |
|---|---|---|---|
| **Mean T4NSS at the baseline** | n | 68 | 70 |
| | Mean | 8.65 | 8.64 |
| | SD | 0.427 | 0.450 |
| | Median | 8.67 | 8.67 |
| | 25^{th} %ile, 75^{th} %ile | 8.33, 9.00 | 8.33, 9.00 |
| | Min, max | 7.7, 9.3 | 7.3, 9.3 |
| **Mean T4NSS over the treatment period** | n | 68 | 70 |
| | Mean | 3.92 | 3.33 |
| | SD | 2.403 | 2.165 |
| | Median | 3.56 | 3.04 |
| | 25th %ile, 75th %ile | 2.18, 5.64 | 1.60, 4.88 |
| | Min, max | 0.1, 10.5 | 0.00, 9.7 |
| **Change from the baseline to mean T4NSS over the treatment period** | n | 68 | 70 |
| | Mean | -4.73 | -5.31 |
| | SD | 2.422 | 2.189 |
| | Median | -5.03 | -5.66 |
| | 25th %ile, 75th %ile | -6.40, -3.19 | -7.03, -3.45 |
| | Min, max | -8.9, 2.5 | -9.2, 0.4 |
| | LS Mean | **-4.73** | **-5.32** |
| | 95% CI | -5.28, -4.18 | -5.86, -4.78 |
| **Groups difference** | Mean difference | - | **-6.8%** |
| | LS mean difference | - | **-0.59** |
| | 95% CI | - | -1.36, 0.18 |
| | *P value* | - | 0.1323 |

| | | | |
|---|---|---|---|
| CI, confidence interval; ITT, intention-to-treat; LS, least squares; Max, maximum, Min, minimum; SD, standard deviation; T4NSS, total four nasal symptom score. | | | |

Therefore, the difference in the mean change showed a trend that the test product (rupatadine 10 mg plus montelukast 10 mg) is superior, with a -6.8% mean difference, in reducing T4NSS compared to the reference product (rupatadine *10 mg plus placebo*) in patients having T4NSS ≥ 48 to < 57 at screening.

### 3.2. Change from baseline (as per investigator) of the Mean T4NSS over the treatment period (ANCOVA analysis, subjects from Spain and Poland, ITT population)

### 3.2.1. Spain and Poland

Changes from the baseline of the mean T4NSS over the treatment period for patients enrolled in phase 2 study from Spain and Poland were analysed/extracted.

In patients enrolled in phase 2 study from Spain and Poland, the least squares (LS) mean change was -5.33 (95% CI -5.94 to -4.71) in the reference product group, and -5.97 (95% CI -6.56 to -5.37) in the test product group. This demonstrates a difference of -0.64 (95% CI -1.24 to -0.04) in the mean change for the test product compared to the reference product. See below Tables 13 and 14.

**Table 13: Type III Model ANOVA for Change from baseline (as per investigator) of the Mean T4NSS over the treatment period. ANCOVA analysis, subjects from Spain and Poland, ITT population**

| **Source** | **Degrees of freedom** | **Sum of squares** | **Mean Square** | **F Value** | **p-value** |
|---|---|---|---|---|---|
| Mean T4NSS at Baseline | 1 | 46.972 | 46.972 | 10.63 | 0.0013 |
| Treatment | 1 | 19.615 | 19.615 | 4.44 | 0.0365 |
| Site | 13 | 225.271 | 17.329 | 3.92 | <0.0001 |

**Table 14: Change from baseline (as per investigator) of the Mean T4NSS over the treatment period. ANCOVA analysis, subjects from Spain and Poland, ITT population**

| **Variable** | **Statistics** | **Rupatadine 10 mg + Placebo (N=98)** | **Rupatadine 10 mg + Montelukast 10 mg (N=100)** |
|---|---|---|---|
| **Mean T4NSS at the baseline** | n | 98 | 100 |
| | Mean | 9.59 | 9.69 |
| | SD | 1.150 | 1.218 |
| | Median | 9.33 | 9.50 |
| | 25^{th} %ile, 75^{th} %ile | 8.50, 10.50 | 8.67, 10.58 |
| | Min, max | 7.7, 12.0 | 7.3, 12.0 |
| **Mean T4NSS over the treatment period** | n | 98 | 100 |
| | Mean | 3.99 | 3.40 |
| | SD | 2.449 | 2.266 |
| | Median | 3.61 | 3.07 |
| | 25th %ile, 75th %ile | 2.26, 5.23 | 1.85, 4.17 |
| | Min, max | 0.1, 11.9 | 0.0, 10.9 |
| **Change from the baseline to mean T4NSS over the treatment period** | n | 98 | 100 |
| | Mean | -5.60 | -6.29 |
| | SD | 2.526 | 2.220 |
| | Median | -5.80 | -6.44 |
| | 25th %ile, 75th %ile | -7.28, -4.61 | -7.85, -5.17 |
| | Min, max | -10.5, 2.5 | -10.4, 0.4 |
| | LS Mean | **-5.33** | **-5.97** |
| | 95% CI | -5.94, -4.71 | -6.56, -5.37 |
| **Groups difference** | Mean difference | - | **-6.5%** |
| | LS mean difference | - | **-0.64** |
| | 95% CI | - | -1.24, 0.04 |
| | *P value* | - | 0.0365 |

| | | | |
|---|---|---|---|
| CI, confidence interval; ITT, intention-to-treat; LS, least squares; Max, maximum, Min, minimum; SD, standard deviation; T4NSS, total four nasal symptom score. | | | |

Therefore, the difference in the mean change showed a trend that the test product (rupatadine 10 mg plus montelukast 10 mg) is superior, with a -5.97 difference, in reducing T4NSS compared to the reference product (rupatadine 10 mg plus placebo).

### 3.2.2 Poland only

In patients enrolled in phase 2 study from Poland (N=150), the least squares (LS) mean difference was -0.6751 (95% CI -1.3111 to -0.0390) in favor of the test product. Therefore, that difference showed a trend that the test product (rupatadine 10 mg plus montelukast 10 mg) is superior in reducing all T4NSS symptoms compared to the reference product (rupatadine 10 mg plus placebo).

### 3.3. Change from Day 7 of the Mean T4NSS over 7 days of treatment (from Day 8 to Day 14) (ANCOVA analysis, ITT population)

Changes from Day 7 of the Mean T4NSS over 7 days of treatment (i.e. from Day 8 to Day 14) were analysed/extracted from phase 2 study.

In this sub-analysis, baseline was set up at Day 7 of treatment - to gather information on the change of the mean T4NSS from Day 8 to Day 14 of treatment. The least squares (LS) mean change was -0.44 (95% CI -0.77 to -0.11) in the reference product group, and -0.52 (95% CI -0.84 to -0.20) in the test product group. This demonstrates a difference of -0.08 (95% Cl -0.41 to 0.25) in the LS mean difference for the test product compared to the reference product. See below Tables 15 and 16.

**Table 15: Type III Model ANOVA for Change from Day 7 of the Mean T4NSS over 7 days of treatment (from Day 8 to Day 14) (ANCOVA analysis, ITT population)**

| **Source** | **Degrees of freedom** | **Sum of squares** | **Mean Square** | **F Value** | **p-value** |
|---|---|---|---|---|---|
| Mean T4NSS at Baseline | 1 | 156.548 | 156.548 | 79.47 | <.0001 |
| Treatment | 1 | 0.411 | 0.411 | 0.21 | 0.6482 |
| Site | 17 | 106.059 | 6.239 | 3.17 | <.0001 |

**Table 16: Change from Day 7 of the Mean T4NSS over 7 days of treatment (from Day 8 to Day 14) (ANCOVA analysis, ITT population)**

| **Variable** | **Statistics** | **Rupatadine 10 mg + Placebo (N=143)** | **Rupatadine 10 mg + Montelukast 10 mg (N=147)** |
|---|---|---|---|
| **Final T4NSS at Day 7 of treatment** | n | 143 | 147 |
| | Mean | 5.15 | 4.60 |
| | SD | 2.993 | 2.866 |
| | Median | 5.00 | 4.00 |
| | 25^{th} %ile, 75^{th} %ile | 2.50, 7.50 | 2.50, 7.00 |
| | Min, max | 0.0, 12.0 | 0.0, 11.5 |
| **Mean T4NSS from Day 8 to Day 14 of treatment** | n | 143 | 147 |
| | Mean | 4.48 | 4.03 |
| | SD | 2.802 | 2.683 |
| | Median | 4.36 | 3.71 |
| | 25th %ile, 75th %ile | 2.07, 6.93 | 1.86, 6.14 |
| | Min, max | 0.0, 11.9 | 0.0, 10.5 |
| **Change from Day 7 in T4NSS to mean from Day 8 to Day14 of treatment** | n | 143 | 147 |
| | Mean | -0.67 | -0.57 |
| | SD | 1.672 | 1.557 |
| | Median | -0.39 | -0.29 |
| | 25th %ile, 75th %ile | -1.36, 0.21 | -1.29, 0.36 |
| | Min, max | -5.6, 7.9 | -7.4, 3.6 |
| | LS Mean | **-0.44** | **-0.52** |
| | 95% CI | -0.77, -0.11 | -0.84, -0.20 |
| **Groups difference** | Mean difference | - | **-0.6%** |
| | LS mean difference | - | **-0.08** |
| | 95% CI | - | -0.41, 0.25 |
| | *P value* | - | 0.6482 |

| | | | |
|---|---|---|---|
| CI, confidence interval; ITT, intention-to-treat; LS, least squares; Max, maximum, Min, minimum; SD, standard deviation; T4NSS, total four nasal symptom score. | | | |

## Claims

1. A pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 8 as calculated by a single T4NSS measurement evaluation.

2. The pharmaceutical combination for use according to claim 1, wherein the four-component nasal symptom score (T4NSS) of ≥ 8 is calculated by the mean of two T4NSS measurement evaluations over a period of one day.

3. A pharmaceutical combination comprising rupatadine or a pharmaceutically acceptable salt thereof and montelukast or a pharmaceutically acceptable salt thereof for use in the treatment of seasonal allergic rhinitis (SAR) in a patient, wherein the patient is defined by a four-component nasal symptom score (T4NSS) of ≥ 48 as calculated by the summation of six T4NSS measurement evaluations.

4. The pharmaceutical combination for use according to claim 3, wherein the four-component nasal symptom score (T4NSS) of ≥ 48 is calculated by the summation of six T4NSS measurement evaluations, wherein the measurements are performed up to twice a day over a period of four to seven days and include at least two non-consecutive measurements.

5. The pharmaceutical combination for use according to any one of claims 3 or 4, wherein the patient is defined by a four-component nasal symptom score (T4NSS) of ≥ 48 to < 60 as calculated by the summation of six T4NSS measurement evaluations.

6. The pharmaceutical combination for use according to any one of claims 1 to 5, wherein the seasonal allergic rhinitis is moderate seasonal allergic rhinitis or severe seasonal allergic rhinitis.

7. The pharmaceutical combination for use according to any one of claims 1 to 6, wherein the patient has mild or moderate bronchial asthma.

8. The pharmaceutical combination for use according to any one of claims 1 to 6, wherein the patient does not have mild or moderate bronchial asthma.

9. The pharmaceutical combination for use according to any one of claims 1 to 6 or 8, wherein the patient does not have bronchial asthma.

10. The pharmaceutical combination for use according to any one of claims 1 to 9, wherein the treatment of SAR is symptomatic treatment.

11. The pharmaceutical combination for use according to claim 10, wherein symptomatic treatment is provided by the treatment of one or more symptoms selected from:
• nasal blockade / congestion;
• rhinorrhea (running nose);
• nasal itching;
• sneezing;
• pharyngeal itching;
• tearing; and
• conjunctival redness / ocular pruritus

12. The pharmaceutical combination for use according to claim 10 or 11, wherein symptomatic treatment is provided by the treatment of:
• nasal blockade / congestion, rhinorrhea (running nose), nasal itching and/or sneezing; and/or
• pharyngeal itching, tearing and/or conjunctival redness / ocular pruritus.

13. The pharmaceutical combination for use according to any one of claims 10 to 12, wherein symptomatic treatment is provided by the treatment of sneezing and/or rhinorrhea.

14. The pharmaceutical combination for use according to any one of claims 1 to 13, wherein rupatadine and montelukast or pharmaceutically acceptable salts thereof are provided in an oral dosage form adapted for separate, sequential or simultaneous administration.

15. The pharmaceutical combination for use according to claim 14, wherein the combination is administered once daily.

16. The pharmaceutical combination for use according to claim 15, wherein the combination is administered once daily for at least 7 days, 14 days, 21 days, 28 days, 35 days, 42 days, 49 days, 56 days, 63 days, 70 days, 77 days or 84 days.

17. The pharmaceutical combination for use according to claim 15, wherein the combination is administered once daily for at least 1 month, 2 months, 3 months, 4 months, 5 months or 6 months.

18. The pharmaceutical combination for use according to any one of claims 14 to 17, wherein rupatadine and montelukast or pharmaceutically acceptable salts thereof are provided in separate oral dosage forms or are provided as a fixed-dose composition in a single oral dosage form.

19. The pharmaceutical combination for use according to claim 18, wherein the fixed-dose composition is a tablet, for example a monolayer or multilayer tablet.

20. The pharmaceutical combination for use according to claim 19, wherein the fixed-dose composition further comprises one or more pharmaceutically acceptable excipients, for example a diluent, binder, disintegrant, lubricant, glidant, antioxidant, colourant and/or flavouring agent.

21. The pharmaceutical combination for use according to claim 20, wherein the fixed-dose composition further comprises a diluent, binder, disintegrant and lubricant, wherein the total amount of diluents is of from 20 wt% and 90 wt% w/w, the total amount of binders is of from 2 and 14 wt%, the total amount of disintegrants is of from 1 and 15 wt % and the total amount of lubricants is of from 0.2 and 2.0 wt%.

22. The pharmaceutical combination for use according to any one of claims 14 to 21, wherein the oral dosage form comprises rupatadine or a pharmaceutically acceptable salt thereof in an amount of from 0.5 to 22 mg, expressed as weight of rupatadine free base.

23. The pharmaceutical combination for use according to any one of claims 14 to 22, wherein the oral dosage form comprises montelukast or a pharmaceutically acceptable salt thereof in an amount of from 1 to 22 mg, expressed as weight of montelukast free acid.

24. The pharmaceutical combination for use according to any one of claims 14 to 23, comprising of from 11 to 14 mg rupatadine fumarate and of from 9 to 11 mg montelukast sodium.

25. The pharmaceutical combination for use according to any one of claims 14 to 24, wherein symptomatic treatment is provided by the treatment of sneezing and/or rhinorrhea following once-daily treatment with the combination for 7 days or 14 days.

26. The pharmaceutical combination for use according to any one of claims 1 to 25, wherein the patient is of from 15 to 18 years of age, 19 to 64 years of age, or ≤ 65 years of age.
